(19) Europäisches Patentamt
European Patent Office
Office européen des brevets

(11) **EP 4 737 452 A1**

(12) **EUROPEAN PATENT APPLICATION**
published in accordance with Art. 153(4) EPC


(43) Date of publication:
**06.05.2026 Bulletin 2026/19**

(21) Application number: **24832520.1**

(22) Date of filing: **28.06.2024**

(51) International Patent Classification (IPC):
***C07D 421/12*** (2006.01) ***A61K 31/095*** (2006.01)
***A61K 31/517*** (2006.01) ***A61K 31/5377*** (2006.01)
***A61K 31/675*** (2006.01) ***A61K 31/695*** (2006.01)
***C07D 487/04*** (2006.01) ***C07D 471/04*** (2006.01)
***C07D 491/056*** (2006.01) ***C07D 421/14*** (2006.01)

(52) Cooperative Patent Classification (CPC):
**A61K 31/095; A61K 31/517; A61K 31/5377; A61K 31/675; A61K 31/695; C07D 421/12; C07D 421/14; C07D 471/04; C07D 487/04; C07D 491/056**

(86) International application number:
**PCT/KR2024/009140**

(87) International publication number:
**WO 2025/005744 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.06.2023 KR 20230084377**
**17.06.2024 KR 20240078038**



(71) Applicant: **Aigen Sciences Inc.**
**Seoul 02841 (KR)**

(72) Inventors:
- **LEE, Kwang-Ok**
  **Seoul 04778 (KR)**
- **LEE, Ho-Jeong**
  **Seoul 04778 (KR)**
- **KIM, Sunkyu**
  **Seoul 04778 (KR)**
- **KIM, Jihye**
  **Seoul 04778 (KR)**
- **PARK, Sejeong**
  **Seoul 04778 (KR)**
- **LEE, Sanghoon**
  **Seoul 04778 (KR)**
- **YOO, Kiwoong**
  **Seoul 04778 (KR)**
- **YOO, Hyerim**
  **Seoul 04778 (KR)**

(74) Representative: **Germain Maureau**
**12, rue Boileau**
**69006 Lyon (FR)**


(54) **NOVEL PYRIMIDINE DERIVATIVES**


(57) The present invention provides a compound defined by chemical formula 1 or a tautomer thereof, and pharmaceutically acceptable salts thereof. The compound of the present invention can inhibit the activity of ubiquitin-specific protease 1.


EP 4 737 452 A1

[Chemical Formula 1]

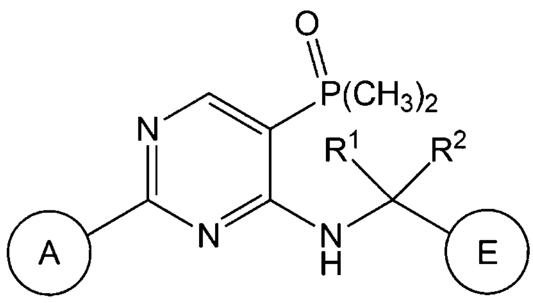

【FIG. 1】

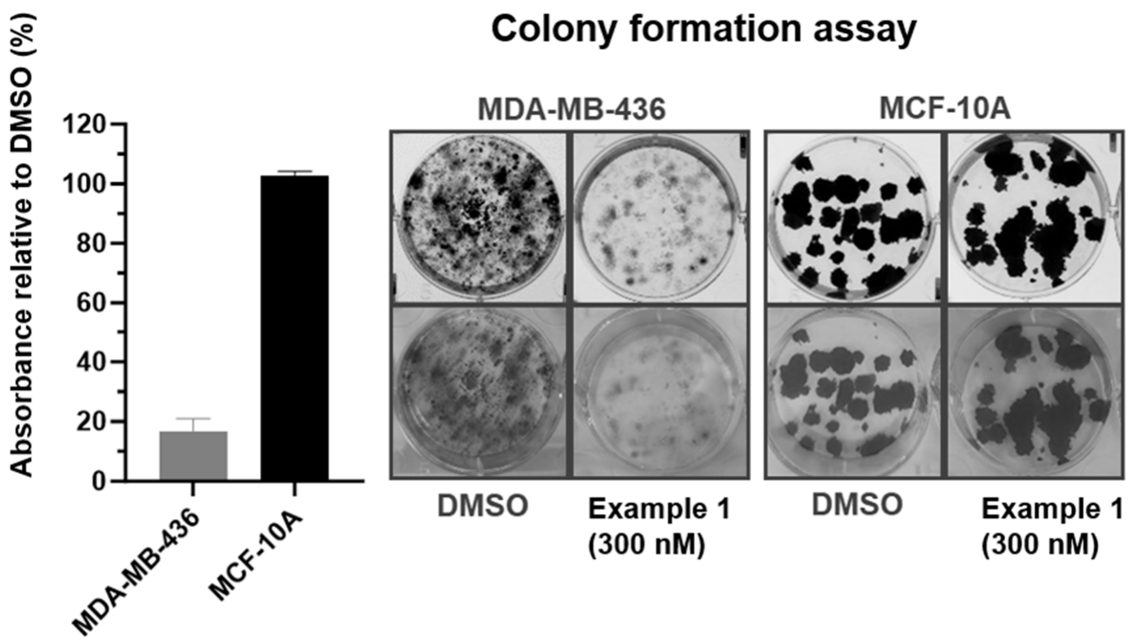

## Description

[TECHNICAL FIELD]

The present invention relates to small organic compounds exhibiting an inhibitory activity against biological enzymes. In addition, the present invention relates to a pharmaceutical composition including the small organic compounds.

[BACKGROUND ART]

Ubiquitin is a small protein composed of 76 amino acids and plays an important role in the regulation of intracellular protein function. Ubiquitination and de-ubiquitination are enzymatically mediated processes in which ubiquitin is covalently attached to and removed from target protein, respectively. Ubiquitination and de-ubiquitination are involved in important functions such as the regulation of the cell cycle, apoptosis, the marking of membrane proteins like receptors for removal, and the regulation of DNA transcription and repair. Proteins in cells become targets for proteasomal degradation when they are tagged with three or more ubiquitin molecules. De-ubiquitination serves to recycle ubiquitin and restores the function of proteins from which ubiquitin has been removed. There are approximately 95 distinct deubiquitinating enzymes present in human cells. Among these, ubiquitin-specific protease 1 (USP1) is known to be involved in the repair of DNA damage caused by agents that induce DNA crosslinks, such as ionizing radiation, ultraviolet light, and cisplatin. USP1 forms a complex with USP1 associated factor 1 (UAF1) and is activated by UAF1 (Cohn et al., 2007, Mol Cell 28:786-797).

Ubiquitin-specific protease 1 (USP1) is known to be overexpressed in various cancer cells. Therefore, selective inhibition of USP1 can lead to cancer cell death by suppressing DNA damage repair in these cells. Recently, USP1 has been identified through CRISPR-Cas9 screening as a novel synthetic lethality target in BRCA1-mutant cancers, and USP1 has also been shown to serve as a synthetic lethal target in BRCA2-mutant cancers. Furthermore, USP1 inhibitors have been reported to exhibit potent anticancer activity against BRCA-mutant cancers when used in combination with PARP inhibitors. Additionally, recent studies have shown that USP1 inhibitors demonstrate therapeutic potential for diabetes by suppressing pancreatic beta cell apoptosis. USP1 plays a critical role in adipogenesis by regulating the ubiquitination of the transcription factor C/EBPβ, and selective inhibitor of USP1 shows its potential as a treatment for metabolic disorders including obesity. Therefore, USP1 inhibitors could be developed as therapeutic agents not only for cancer including BRCA-mutant cancers, but also for metabolic diseases such as diabetes and obesity.

[DISCLOSURE]

[TECHNICAL PROBLEM]

The technical object of the present invention is to provide a novel small organic molecule compound capable of inhibiting an activity of USP1 as a deubiquitinating enzyme.

[TECHNICAL SOLUTION]

In one aspect of the present invention, a compound defined by chemical formula 1 having an inhibitory activity against USP1 or a tautomer thereof, and a pharmaceutically acceptable salt thereof are provided.

【Chemical Formula 1】

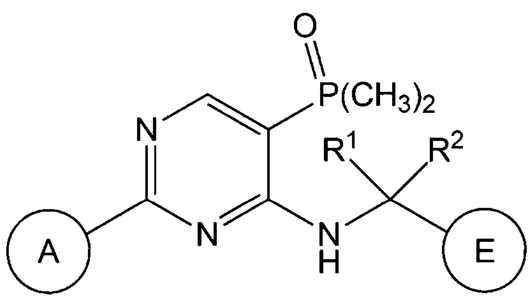

In Chemical Formula 1, $R^1$ and $R^2$ are each independently hydrogen, deuterium, halogen, $C_{1-4}$ alkyl, -O-$C_{1-4}$

alkyl, -CN or -NHC$_{1-4}$ alkyl;

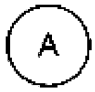

are rings independently selected from the group consisting of substituted or non-substituted C$_{6-10}$ aryl, substituted or non-substituted 5- to 6-membered heteroaryl, substituted or non-substituted C$_{5-10}$ cycloalkyl and substituted or non-substituted cubanyl;

wherein the substituted 5- to 6-membered heteroaryl represents a ring in which at least one hydrogen of the ring is substituted with a functional group independently selected from the group consisting of C$_{1-6}$ alkyl, C$_{1-6}$ alkyl substituted with deuterium, haloC$_{1-6}$ alkyl, C$_{3-8}$ cycloalkyl, -OC$_{1-4}$ alkyl, -OC$_{1-4}$ alkyl substituted with deuterium, haloC$_{1-4}$ alkyloxy and halogen, and

wherein the substituted C$_{6-10}$ aryl, substituted C$_{5-10}$ cycloalkyl, substituted C$_{5-10}$ cycloalkyl and substituted cubanyl represents a ring in which at least one hydrogen of the ring is substituted with a functional group independently selected from the group consisting of C$_{1-6}$ alkyl, C$_{1-6}$ alkyl substituted with deuterium, haloC$_{1-6}$ alkyl, C$_{3-8}$ cycloalkyl, -OC$_{1-4}$ alkyl, -OC$_{1-4}$ alkyl substituted with deuterium, haloC$_{1-4}$ alkyloxy, halogen, 5- to 6-membered heteroaryl, and functionality bearing 5- to 6-membered heteroaryl,

wherein the functionality bearing 5- to 6-membered heteroaryl represents a heteroaryl ring in which one or more of the ring hydrogens thereof is substituted with C$_{1-6}$ alkyl, haloC$_{1-6}$ alkyl or C$_{3-8}$ cycloalkyl.

In one embodiment of the present invention, a compound defined by Chemical Formula 2 or a tautomer thereof and a pharmaceutically acceptable salt thereof are provided.

【Chemical Formula 2】

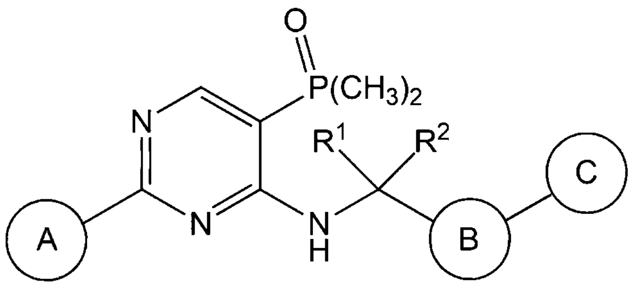

In Chemical Formula 2, R$^1$ and R$^2$ are each independently hydrogen, deuterium, halogen, C$_{1-4}$ alkyl, -O-C$_{1-4}$ alkyl, -CN or -NHC$_{1-4}$ alkyl;

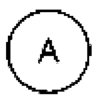

is selected from the group consisting of substituted or non-substituted C$_{6-10}$ aryl, substituted or non-substituted 5- to 6-membered heteroaryl, substituted or non-substituted C$_{5-10}$ cycloalkyl and substituted or non-substituted cubanyl;

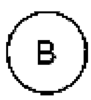

is selected from the group consisting of substituted or non-substituted phenylene, substituted or non-substituted 5- to 6-membered heteroarylene, substituted or non-substituted C$_{5-10}$ bicycloalkylene, substituted or non-substituted adamantylene, substituted or non-substituted norbornenyl and substituted or non-substituted cubanylene;

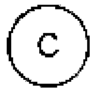

is selected from substituted or non-substituted phenyl and substituted or non-substituted 5- to 6-membered heteroarylene.

In compound of Chemical Formula 1, substituted $C_{6-10}$ aryl, substituted phenylene, substituted 5- to 6-membered heteroaryl, substituted 5- to 6-membered heteroarylene, substituted $C_{5-10}$ cycloalkyl, substituted $C_{5-10}$ bicycloalkyl, substituted $C_{5-10}$ bicycloalkylene, substituted adamantylene, substituted norbornenylene, substituted cubanyl and substituted cubanylene refer to groups in which one or more ring hydrogens - that is, those hydrogens bonded to a carbon atom or a heteroatom constituting the ring backbone - of the above-mentioned $C_{6-10}$ aryl, phenylene, 5- to 6-membered heteroaryl, 5- to 6-membered heteroarylene, $C_{5-10}$ cycloalkyl, $C_{5-10}$ bicycloalkyl, $C_{5-10}$ bicycloalkylene, adamantylene, norbornenylene, cubanyl and cubanylene, is optionally substituted with a substituent, wherein such substituent is selected from the group consisting of $C_{1-6}$ alkyl, $C_{1-6}$ alkyl substituted with deuterium, halo$C_{1-6}$ alkyl, $C_{3-8}$ cycloalkyl, -O$C_{1-4}$ alkyl, -O$C_{1-4}$ alkyl substituted with deuterium, halo$C_{1-4}$ alkyloxy and halogen.

Another aspect of the present invention provides a pharmaceutical composition for the treatment of cancer, comprising the compound described above or a tautomer thereof, or a pharmaceutically acceptable salt thereof, in a therapeutically effective amount, and a pharmaceutically acceptable excipient.

In yet another aspect, a method for the treatment of cancer is provided. The method comprises administering to a patient a therapeutically effective amount of the compound of the present invention, e.g., a compound defined by Chemical Formula 1, or a pharmaceutical composition comprising the compound of the present invention.

[ADVANTAGEOUS EFFECTS]

The compounds of the present invention can inhibit USP1 activity. Furthermore, the compounds of the present invention have selectivity to exhibit a synthetic lethal effect in conjunction with BRCA activity. The compounds of the present invention are useful for the treatment of cancer.

[BRIEF DESCRIPTION OF DRAWINGS]

Fig. 1 is an experimental result showing that a synthetic lethal phenotype was expressed when the compound of the present invention was added to BRCA1 wild-type cell line and BRCA1 mutant cell line with reduced BRCA1 activity.

[MODE FOR INVENTION]

Hereinafter, embodiments of the present invention will be described in detail. Prior to this, the terms and words used in this specification and claims should not be construed as limited to their conventional or dictionary meanings. Based on the principle that inventors can appropriately define the concepts of terms to best explain their inventions, they should be interpreted in a way that aligns with the technical concept of the present invention.

Therefore, the embodiments described in this specification are merely examples presented to aid understanding of the present invention and do not fully represent the technical concept of the present invention. Therefore, it should be understood that various equivalents and modifications may exist at the time of filing.

Unless otherwise defined, all technical terms used in this specification have the same meaning as commonly understood by those skilled in the art. While preferred methods and samples are described herein, similar or equivalent methods are also included within the scope of the present invention. Numerical values described herein are assumed to include the meaning of "about," even if not explicitly stated. The numerical range indicated using the term "to" in this specification includes the ranges that include the numerical values described before and after the term "to" as the lower and upper limits, respectively.

<u>Definition of terms</u>

As used herein, the term "ubiquitin-specific-processing protease 1 (USP1)" or "USP-1" refers to a cysteine protease having deubiquitinating activity and forming a complex with UAF1. As used herein, the term "USP1" includes not only the full-length polypeptide, but also forms that are generated from processing of the protein within a cell (e.g., processing of cleaving signal peptide portion, etc.). Furthermore, in the present specification, the term "USP1" includes naturally occurring variants of USP1, such as splicing variants and allelic variants. While USP1 is generally described with

reference to human USP1 in the present specification, this is not intended to be limited to proteins derived exclusively from humans. The sequence of human USP1 can be referred in the protein sequence database as UniProt No. 094782.

As used herein, the term "suppression of USP1" or "inhibition of USP1" or "suppression of USP1 activity" or "inhibition of USP1 activity" or the term "USP1 suppressor" or "USP1 inhibitor" for the compounds refers to action of reducing the deubiquitinating activity of USP1. For example, "USP1 suppressor" or "USP1 inhibitor" is a substance that has an action of reducing the measured signal of USP1 deubiquitinating activity by at least about 10%, at least about 20%, at least about 30%, at least about 40%, at least about 50%, at least about 60%, at least about 70%, at least about 80%, at least about 90%, or at least about 95%, when measured under comparable conditions with a negative control (e.g., a blank).

As used herein, the term "deubiquitinating activity of USP1" or "USP1 activity" refers to the action of USP1 in removing ubiquitin from ubiquitinated substrates. Deubiquitinating activity can be measured either directly or indirectly, by in vitro or in vivo experimental methods, and for example, by assays for detecting the de-ubiquitination of natural target substrate such as FANCD2-Ub or PCNA-Ub that is natural target of USP1. As different method thereof, when USP1 hydrolyzes an amide bond in reagent of ubiquitin conjugated to a fluorophore via an amide bond, the increase in fluorescence signal intensity caused by the release of a fluorophore can be measured, for example, by using ubiquitin linked to the fluorophore such as rhodamine 110 or 7-amido-4-methylcoumarin (AMC). If the concentration of deubiquitinated FANCD2, PCNA, rhodamine 110, or AMC in the presence of USP1 increases by 10% or more over the concentration of deubiquitinated FANCD2, PCNA, rhodamine 110, or AMC in a control sample (e.g., another sample from the same tissue or another aliquot of the same cell sample), a USP1 polypeptide is considered to have deubiquitinating activity.

Those skilled in the art will appreciate that some of the compounds of the present invention may exist in more than one tautomeric form. Since a single chemical structural formula can only represent a single tautomeric form, it is well understood that when a compound is referred to by a single structural formula for convenience, the structural formula encompasses all tautomeric variations of the compound. Depending on the compound, one tautomer may be present primarily, a mixture of multiple tautomers may exist at room temperature, or a single tautomer may be isolated. Examples of tautomers include those between the pyridone and hydroxypyridine forms, and those between the keto and enol forms.

As used herein, the prefix of "$C_{x-y}$" or "$C_x$-$C_y$" (where x and y are natural numbers) is used before a functional group and refers to the number of carbon atoms that the functional group has in its backbone. For example, a $C_{1-4}$ alkyl group refers to an alkyl group having 1 to 4 carbon atoms, and a $C_{1-3}$ alkyl group refers to an alkyl group having 1 to 3 carbon atoms, respectively. For example, a $C_1$-$C_4$ alkoxy group includes methoxy, ethoxy, *n*-propoxy, *i*-propoxy, *n*-butoxy, *sec*-butoxy, and *t*-butoxy. For example, a $C_3$-$C_6$ cycloalkyl group includes cyclopropyl, cyclobutyl, cyclopentyl, or cyclohexyl groups. In addition, in the present specification, when the number of elements forming the ring or the number of rings is indicated in front of a cyclic functional group or cyclic compound (including both carbon rings and heterocycles), the ring is a ring formed of the corresponding number of elements, i.e., carbon and/or heteroatoms, or a structure having the corresponding number of rings. For example, a 6-membered cyclic heteroaryl refers to a heteroaryl group in which the number of ring-forming atoms is 6, comprising one or more heteroatoms together with carbon. Also, for example, a 4-6 membered monocyclic heteroaryl refers to a monocyclic aromatic ring that formed of only one ring rather than a fused ring, bridged ring, or spirocyclic compound, and the sum of the number of carbons and heteroatoms forming the ring backbone is 4 to 6, such as pyrrolyl, pyridinyl, imidazolyl, isoxazolyl, thiophenyl, thiazolyl, and the like.

Unless otherwise specified herein, when a functional group is linked to another part of a compound by a bond, the linkage may be via any atom of the functional group, provided that it is a suitable atom. For example, when referring to a propyl group, both prop-1-yl and prop-2-yl are included.

As used herein, the term "halogen" refers to an atom selected from fluorine, chlorine, bromine, and iodine.

As used herein, the term "$C_{1-6}$ alkyl" refers to a monovalent saturated hydrocarbon group with the chemical formula of $C_nH_{2n+1}$, where n is a natural number from 1 to 6. $C_{1-6}$ alkyl group encompasses any linear or branched saturated hydrocarbon group containing 1 to 6 carbon atoms, such as *n*-propyl, *i*-propyl, 2-methyl-ethyl, *sec*-butyl, *tert*-butyl, and the like.

As used herein, the term "$C_{1-6}$ alkylene" refers to a divalent saturated hydrocarbon group with the chemical formula of $C_nH_{2n}$, where n is a natural number from 1 to 6.

As used herein, the term "cycloalkyl" refers to a monovalent saturated hydrocarbon group containing one or more carbon rings. A monocyclic cycloalkyl group containing only one carbon ring has the chemical formula of $C_nH_{2n-1}$. Examples of monocyclic $C_{3-6}$ cycloalkyl groups include cyclopropyl, cyclobutyl, cyclopentyl, and cyclohexyl.

As used herein, the term "bicyclic" compound refers to a carbocyclic compound or heterocyclic compound composed of two rings, which are formed by sharing one or more ring atoms between the two constituent rings. In a bicyclic compound, the atom shared by the two rings is called a bridgehead atom, and bicyclic compounds are divided into three types depending on the number of bridgehead atoms and whether or not there is a direct connection between the bridgehead atoms. A fused bicyclic compound has two bridgehead atoms that are directly connected to each other by a bond. Examples of fused bicyclic compounds include decalin, naphthalene, anthracene, phenanthrene, indole, benzofuran, purine, and quinoline. A cyclic compound with only one bridgehead atom is a spirocyclic compound. A bridged ring compound has two bridgehead atoms that are not directly connected to each other by a bond, but rather have one or more atoms of rings interposed between the two bridgehead atoms. Examples of bridged cyclic compounds include norbornane, 7-oxabicyclo[2.2.1]heptane, and adamantane.

As used herein, fused bicyclic compounds may be expressed by specifying the number of atoms in each of the two rings that constitute the fused bicyclic compound. For example, thienopyridine, benzofuran, indole, and the like may be referred to as fused bicyclic compounds in which a 5-membered ring and a 6-memberd ring are fused. Similarly, naphthalene, chromane, tetrahydroquinoline, quinoline, quinoxaline, pteridine, and the like may be referred to as fused bicyclic compounds in which a 6-membered ring and 6-membered ring are fused.

As used herein, the term "carbocyclyl" refers to a hydrocarbon group having a saturated or unsaturated ring that is not aromatic, with a ring size of 3 to 14 members, and a monovalent group in which the ring backbone is composed of only carbon atoms without any heteroatoms. As used herein, saturated carbocyclyl includes not only a monocyclic functional group, but also a polycyclic saturated carbocyclyl, for example, a bicyclic hydrocarbon group of a fused, bridged, and spiro saturated hydrocarbon ring. For example, some examples of exemplary saturated $C_{3-10}$ carbocyclyl groups include the aforementioned cycloheptyl ($C_7$), cyclooctyl ($C_8$), bicyclo[1.1.1]pentanyl ($C_5$), bicyclo[2.2.1]heptanyl ($C_7$), bicyclo[2.2.2]octanyl ($C_8$), cyclononyl ($C_9$), cyclodecyl ($C_{10}$), decahydronaphthalenyl (decalinyl) ($C_{10}$), adamantyl ($C_{10}$), spiro[4.5]decanyl ($C_{10}$), and the like. Unsaturated carbocyclyl refers to a hydrocarbon ring having one or more double bonds or triple bonds in the ring and is not aromatic. Examples of unsaturated carbocyclyl include cyclopentadienyl, cyclohexenyl, norbornenyl, and the like.

As used herein, the term "aryl" refers to a monovalent hydrocarbon functional group that has an aromatic hydrocarbon ring of 6 to 14 carbon atoms in the ring backbone and may contain one to three fused rings. Examples of aryl include phenyl, naphthyl, anthracenyl, and phenanthrenyl.

As used herein, the term "heteroaryl" refers to a monovalent 5- to 12-membered unsaturated ring that is an aromatic ring, comprising carbon atoms together with one or more heteroatoms selected from nitrogen, oxygen, and sulfur in the ring backbone. The ring system of the heteroaryl may be a single ring, or a double ring or triple ring in the form of a fused ring. The rings containing direct bonds between oxygen and sulfur atoms among the ring-constituting atoms, such as -O-O-, -O-S-, or -S-S-, are excluded from the term of heteroaryl. Some examples of heteroaryl include pyrrolyl, imidazolyl, pyrazolyl, 2-pyridyl, 3-pyridyl, 4-pyridyl, pyrimidyl, triazolyl, quinazolinyl, 2-furanyl, 3-furanyl, benzofuranyl, 2-thienyl, oxazolyl, isothiazolyl, and thiadiazolyl. Heteroaryls may also be defined by their heteroatoms; for example, a heteroaryl in which the heteroatom is nitrogen includes pyrrolyl, imidazolyl, pyrazolyl, and 2-pyridyl, but excludes furanyl and thienyl.

Fused bicyclic heteroaryl groups include 6-membered and 5-membered fused bicyclic heteroaryl groups such as benzimidazolyl, benzofuranyl, benzothiophenyl, isoindolyl, indazolyl, imidazopyridinyl, imidazopyrimidinyl, imidazopyridazinyl, indazolyl, pyrazoleopyridinyl, pyrrolopyrimidinyl, pyrrolopyridinyl, pyrrolopyrazinyl, triazolopyridinyl, triazolopyrimidinyl, purinyl, benzothiazolyl, benzoisothiazolyl, benzothiadiazolyl, benzoxazolyl, benzothiadiazolyl, and the like, and 6-membered and 6-membered fused bicyclic heteroaryl compounds such as benzopyridinyl, benzopyrimidinyl, quinolinyl, isoquinolinyl, phthalazinyl, quinazolinyl, quinoxalinyl, naphthyridinyl, quinolinzinyl, and cinnolinyl.

As used herein, the term "heteroarylene" refers to a divalent aromatic ring derived from the aforementioned heteroaryl, by removing one hydrogen atom.

As used herein, the term "heterocycloalkyl" refers to a non-aromatic ring of a monovalent group in which at least one carbon atom forming the cycloalkyl ring is independently substituted with a heteroatom selected from nitrogen, oxygen, and sulfur. Examples of heterocycloalkyl include pyrrolidinyl, imidazolidinyl, piperidinyl, pyrrolinyl, piperazinyl, morpholinyl, and thiazolidinyl.

As used herein, the term "4- to 6-membered partially-saturated cyclic fused aryl" refers to a monovalent fused bicyclic ring, wherein the first ring of the two constituents rings is an aryl group, i.e., a phenyl group or a substituted phenyl group; and the second ring is a 4- to 6-membered hydrocarbon ring including the two unsaturated ring carbons shared with the first aryl ring, or a heterocyclic ring comprising a heteroatom as a ring-constituting atom, which is a non-aromatic ring as it also includes the saturated bond within its skeleton. The 4- to 6-membered partially-saturated cyclic fused aryl may be connected to the remaining part of the compound through either the first ring (aryl ring) or the second ring (4- to 6-membered partially-saturated ring). Examples of partially-saturated bicyclic aryls include indanyl, tetralinyl, dialinyl, benzopyranyl, tetrahydroquinolinyl, indolinyl, isoindolinyl, oxindolyl, benzodioxanyl, dihydrobenzofuryl, chromanyl, and dihydrobenzimidazolyl.

As used herein, the term "4- to 6-membered partially-saturated cyclic fused heteroaryl" refers to a monovalent fused bicyclic ring, wherein the first ring of the two constituents rings is a monovalent heteroaryl group; and the second ring is a 4- to 6-membered hydrocarbon ring including the two ring-constituting atoms shared with the first heteroaryl ring, or a heterocyclic ring comprising a heteroatom as a ring-constituting atom, which is a non- aromatic as it also includes the saturated bond within the ring skeleton,. The 4- to 6-membered partially saturated cyclic fused hetroaryl may be connected to the remaining part of the compound through either the first ring or the second ring. Examples of partially saturated bicyclic heteroaryls include tetrahydroindolyl, dihydrothienodioxinyl, dihydropyrrolopyridinyl, and dihydropyridooxazinyl.

As used herein, the term "substitution" or "substituted" in front of a functional group, unless otherwise stated in the specification, refers to a structure in which one or more hydrogens of the functional group are replaced by a designated functional group other than hydrogen, assuming that the substituted functional group maintains its normal valence and produces a chemically-stable functional group. In typical cases, the substituent is selected from halogen, CN, OH, $C_{1-6}$ alkyl, $C_{3-6}$ cycloalkyl, $C_{3-14}$ heteroaryl, $C_{1-6}$ alkoxy and $CF_3$.

As used herein, when a specific atom of a substituent constituting a part of a compound is marked with symbol of "  ", it indicates that the substituent is chemically bonded to the remaining part of the compound through that atom.

As used herein, when a bond line is marked with a dotted line such as "--", it indicates that the bond line is an optional element that may or may not be present. For example, the structure of

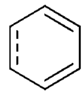

indicates that this structural formula includes benzene and 1,3-cyclohexadiene.

As used herein, the term "pharmaceutically acceptable salt" thereof for a compound refers to a salt that does not impair the desired biological activity of the compound and does not cause inappropriate toxicity, irritation, or allergic reactions when used in contact with human or animal tissues. Pharmaceutically acceptable salts are well known in the art. For example, reference may be made to prior literature such as the paper by Berge et al., J. Pharmaceutical Sciences (1977), Vol. 66, pp. 1-19. Some examples of pharmaceutically acceptable salts include acid addition salts such as hydrochloride, hydrobromide, phosphate, sulfate, perchlorate, acetate, oxalate, maleate, tartrate, citrate, succinate, malonate, adipate, alginate, ascorbate, aspartate, benzoate, benzenesulfonate, and hydrosulfide, and base addition salts such as alkali metal salts, alkaline earth metal salts, ammonium salts, and quaternary ammonium ($N^+(C_{1-4}$ alkyl$)_4$), wherein examples of alkali metal or alkaline earth metal salts include sodium, lithium, potassium, calcium, and magnesium.

Compounds

In one aspect of the present invention, a compound defined by Chemical Formula 1, a tautomer thereof, and pharmaceutically acceptable salts thereof is provided.

【Chemical Formula 1】

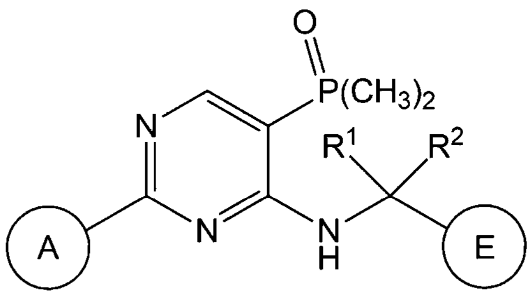

In Chemical Formula 1, $R^1$ and $R^2$ are each independently hydrogen, deuterium, halogen, $C_{1-4}$ alkyl, -O-$C_{1-4}$ alkyl, -CN or -NH$C_{1-4}$ alkyl;

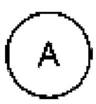 and 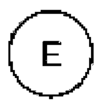

are rings independently selected from the group consisting of substituted or non-substituted $C_{6-10}$ aryl, substituted or non-substituted 5- to 6-membered heteroaryl, substituted or non-substituted $C_{5-10}$ cycloalkyl and substituted or non-substituted cubanyl.

In Chemical Formula 1, the substituted 5- to 6-membered heteroaryl refers to a ring in which at least one of the ring hydrogens is substituted with a functional group independently selected from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, - $OC_{1-4}$ alkyl substituted with deuterium, halo$C_{1-4}$ alkyloxy and halogen, and the substituted $C_{6-10}$ aryl, substituted 5- to 6-membered heteroaryl, substituted $C_{5-10}$ cycloalkyl, substituted $C_{5-10}$ cycloalkyl, and substituted cubanyl refers to one in which at least one hydrogen of the ring is substituted with a substituent. Such substituent may be each independently selected at each occurrence from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, -$OC_{1-4}$ alkyl substituted with deuterium, halo$C_{1-4}$ alkyloxy, halogen, 5- to 6-membered heteroaryl, and functionality bearing 5- to 6-membered heteroaryl. Among these substituents, the functionality bearing 5- to 6-membered heteroaryl refers to one in which at least one hydrogen of the 5- to 6-membered heteroaryl ring is substituted with $C_{1-6}$ alkyl, halo$C_{1-6}$ alkyl, or $C_{3-8}$ cycloalkyl.

In one embodiment of the present invention, the compound of Chemical Formula 1 is a compound having a structure that also satisfies Chemical Formula 2.

【Chemical Formula 2】

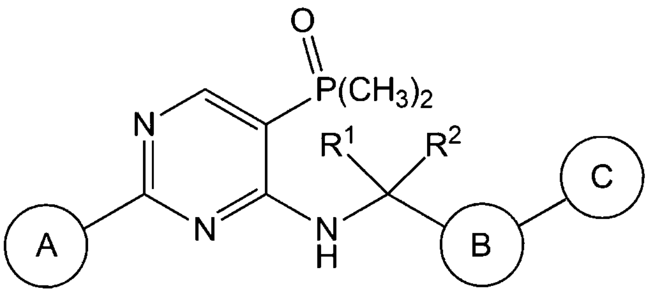

In Chemical Formula 2, $R^1$ and $R^2$ are each independently hydrogen, deuterium, halogen, $C_{1-4}$ alkyl, -O-$C_{1-4}$ alkyl, -CN or -NH$C_{1-4}$ alkyl;

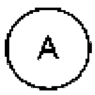

is selected from the group consisting of substituted or non-substituted $C_{6-10}$ aryl, substituted or non-substituted 5- to 6-membered heteroaryl, substituted or non-substituted $C_{5-10}$ cycloalkyl and substituted or non-substituted cubanyl;

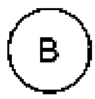

is selected from the group consisting of substituted or non-substituted phenylene, substituted or non-substituted 5- to 6-membered heteroarylene, substituted or non-substituted $C_{5-10}$ bicycloalkylene, substituted or non-substituted adamantylene, substituted or non-substituted norbornenyl and substituted or non-substituted cubanylene;

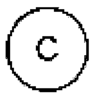

is selected from substituted or non-substituted phenyl and substituted or non-substituted 5- to 6-membered heteroarylene;

wherein the substituted $C_{6-10}$ aryl, substituted phenylene, substituted 5- to 6-membered heteroaryl, substituted 5- to 6-membered heteroarylene, substituted $C_{5-10}$ cycloalkyl, substituted $C_{5-10}$ bicycloalkyl, substituted $C_{5-10}$ bicycloalkylene, substituted adamantylene, substituted norbornenylene, substituted cubanyl and substituted cubanylene refers to one that at least one hydrogen is substituted with a functional group each independently selected from the group

consisting of $C_{1-6}$ alkyl, $C_{1-6}$ alkyl substituted with deuterium, halo$C_{1-6}$ alkyl, $C_{3-8}$ cyclo alkyl, -O$C_{1-4}$ alkyl, -O$C_{1-4}$ alkyl substituted with deuterium, halo$C_{1-4}$ alkyloxy and halogen.

In an embodiment, the compound of Chemical Formula 2 is a compound wherein

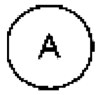

is substituted pyrimidine, substituted pyrazole or substituted pyridine.

In other embodiment, the compound of Chemical Formula 2 is a compound wherein $C_{5-10}$ bicycloalkylene of

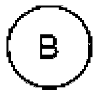

is selected from bicyclo[1.1.1]pentanylene, bicyclo[2.2.1]heptanylene, bicyclo[2.1.1]hexanylene or bicyclo[2.2.2]octanylene.

In other embodiment, the compound of the present invention has a structure represented by Chemical Formula 3.

【Chemical Formula 3】

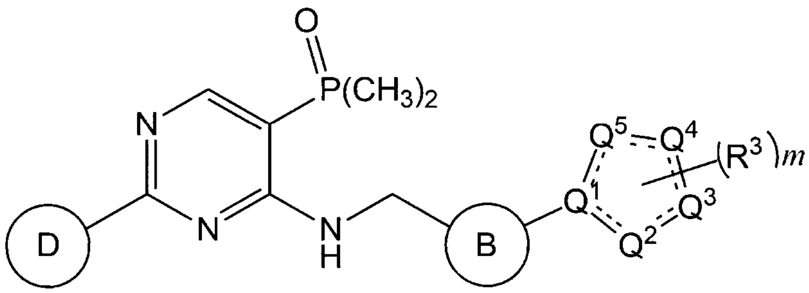

In Chemical Formula 3,

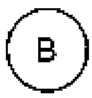

is selected from the group consisting of substituted or non-substituted phenylene, substituted or non-substituted 5- to 6-membered heteroarylene, substituted or non-substituted $C_{5-10}$ bicycloalkylene and substituted or non-substituted cubanylene,

$Q^1$ to $Q^5$ are so selected from the group consisting of C, CH, N and NH as to form any one of the rings represented in Chemical Formula 4 below.

【Chemical Formula 4】

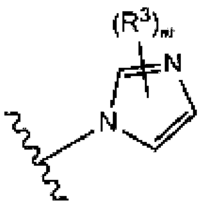 , 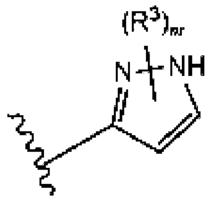 , 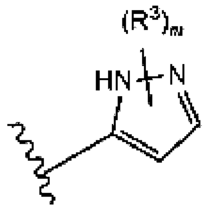 , 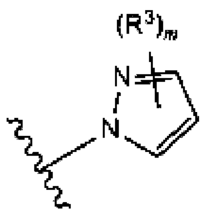 ,

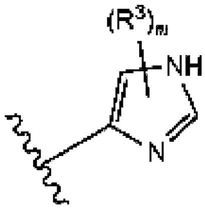 or 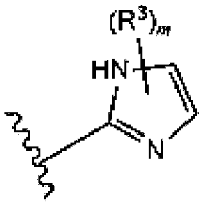

wherein m is an integer of 0 to 3, and $R^3$ is a functional group which substitutes hydrogen at C-H or N-H of 5-membered rings represented by Chemical Formula 4, and up to m hydrogen may be substituted. For each time substituting each hydrogen leading up to the value of m, $R^3$ is each independently selected from the group consisting of $C_{1-6}$ alkyl, $C_{1-6}$ alkyl substituted with deuterium, halo$C_{1-6}$ alkyl, $C_{3-8}$cyclo alkyl, -O$C_{1-4}$ alkyl, -O$C_{1-4}$ alkyl substituted with deuterium, halo$C_{1-4}$ alkyloxy and halogen.

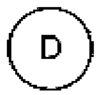

of Chemical Formula 3 is a ring selected from Chemical Formula 5:

【Chemical Formula 5】

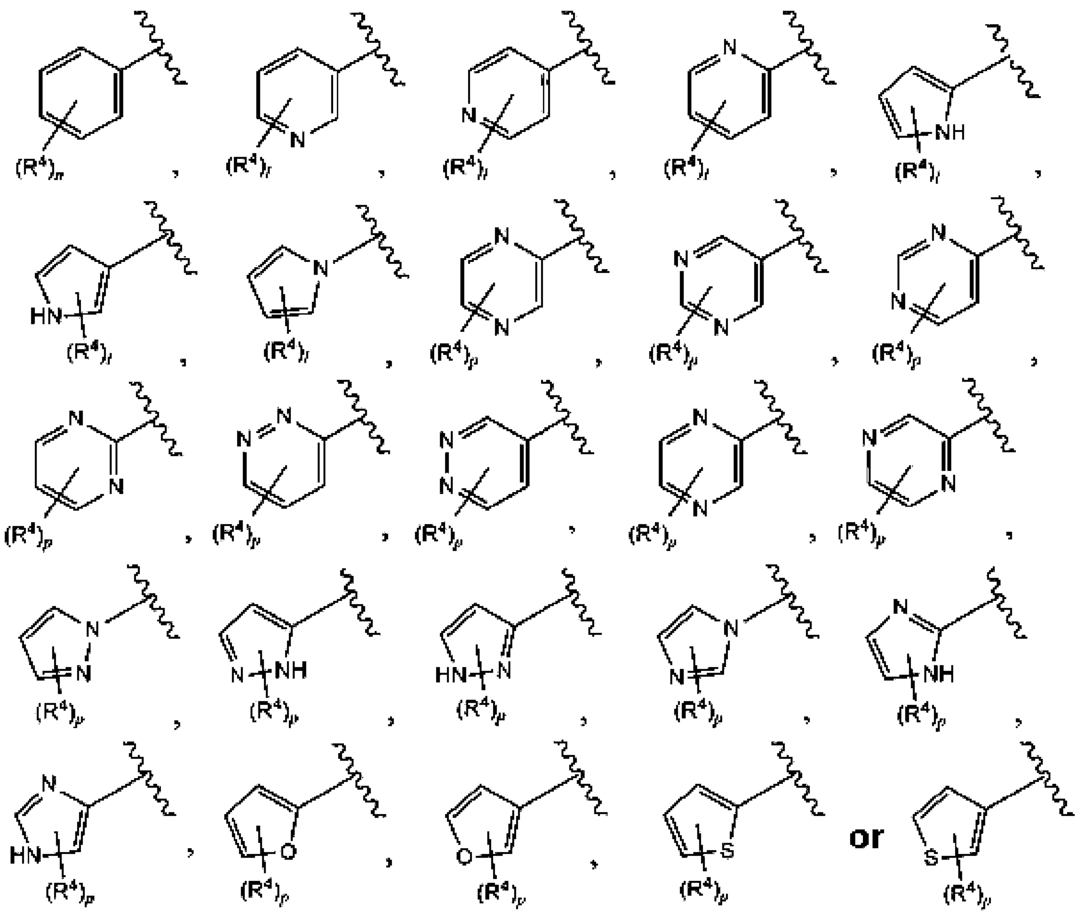

, , , , ,

, , , , ,

, , , , ,

, , , , ,

, , , or

where n is an integer of 0 to 5, *l* is an integer of 0 to 4, *p* is an integer of 0 to 3, and
$R^4$ is a functional group which substitutes hydrogen at C-H or N-H of 5-membered and 6-membered rings represented by Chemical Formula 5, and in each case, up to *n, l* or *p* hydrogen of the rings may be substituted. Such functional group of $R^4$ is independently selected each time for corresponding maximum value of n, l or p, from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, -$OC_{1-4}$ alkyl deuterium substituted, halo$C_{1-4}$ alkyloxy and halogen.

In one specific embodiment, the compound of Chemical Formula 3 is a compound wherein

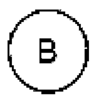

is unsubstituted phenylene or phenylene substituted with halogen.

In another embodiment, the compound of Chemical Formula 3 is a compound having a structure that also satisfies the following Chemical Formula 6.

【Chemical Formula 6】

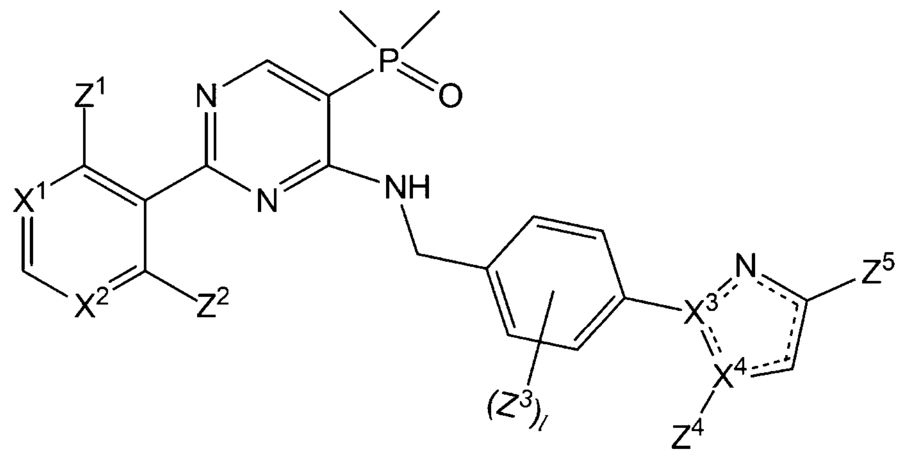

In Chemical Formula 6, $X^1$ and $X^2$ are each independently CH or N, and $X^3$ and $X^4$ are each independently C or N.

$Z^1$, $Z^2$, $Z^4$ and $Z^5$ are substituents each independently selected from the group consisting of hydrogen, $C_{1-6}$ alkyl, $C_{1-6}$ alkyl substituted with deuterium, halo$C_{1-6}$ alkyl, $C_{3-8}$ cycloalkyl, $-OC_{1-4}$ alkyl, $-OC_{1-4}$ alkyl substituted with deuterium, halo$C_{1-4}$ alkyloxy and halogen.

$Z^3$ is a substituent each independently selected at each occurrence up to *l* times from the group consisting of $C_{1-6}$ alkyl, $C_{1-6}$ alkyl substituted deuterium, halo$C_{1-6}$ alkyl, $C_{3-8}$ cycloalkyl, $-OC_{1-4}$ alkyl, $-OC_{1-4}$ alkyl substituted deuterium, halo$C_{1-4}$ alkyloxy and halogen, wherein *l* is an integer of 0 to 4.

In a specific embodiment, the compound of Chemical Formula 6 is a compound in which $Z^3$ is hydrogen or halogen, and $Z^4$ and $Z^5$ are each independently hydrogen, $C_{1-6}$ alkyl, $C_{3-8}$ cycloalkyl or halo$C_{1-6}$ alkyl.

In a more specific embodiment, the compounds of the present invention are the following compounds, stereoisomers, tautomers, solvates or pharmaceutically acceptable salts thereof:

(2-(2-cyclopropyl-6-methoxyphenyl)-4-((4-(1-methyl-4-(trifluoromethyl)-1*H*-imidazol-2-yl)benzyl)amino)pyrimidin-5-yl)dimethylphosphine oxide;
(4'-cyclopropyl-6'-methoxy-4-((4-(1-methyl-4-(trifluoromethyl)-1*H*-imidazol-2-yl)benzyl)amino)-[2,5'-bipyrimidine]-5-yl)dimethylphosphine oxide;
(2-(1-isopropyl-4-methyl-1*H*-pyrazole-5-yl)-4-((4-(1-methyl-4-(trifluoromethyl)-1*H*-imidazol-2-yl)benzyl)amino)pyrimidin-5-yl)dimethylphosphine oxide;
*(2-(2-isopropylpyridine-3-yl)-4-((4-(1-methyl-4-(trifluoromethyl)-1H*-imidazol-2-yl)benzyl)amino)pyrimidin-5-yl)dimethylphosphine oxide;
(4'-cyclopropyl-6'-(methoxy-*d*$_3$)-4-((4-(1-methyl-4-(trifluoromethyl)-1*H*-imidazol-2-yl)benzyl)amino)-[2,5'-bipyrimidine]-5-yl)dimethylphosphine oxide;
(2-(2-(difluoromethoxy)pyridine-3-yl)-4-((4-(1-methyl-4-(trifluoromethyl)-1*H*-imidazol-2-yl)benzyl)amino)pyrimidin-5-yl)dimethylphosphine oxide;
(4'-cyclopropyl-4-((3-fluoro-4-(1-methyl-4-(trifluoromethyl)-1H-imidazol-2-yl)benzyl)amino)-6'-methoxy-[2,5'-bipyrimidine]-5-yl)dimethylphosphine oxide;
(4'-cyclopropyl-4-((4-(1-cyclopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-6'-methoxy-4-((4-(5-methyl-3-(trifluoromethyl)-1*H*-pyrazole-1-yl)benzyl)amino)-[2,5'-bipyrimidine]-5-yl)dimethylphosphine oxide;
(4'-cyclopropyl-6'-methoxy-4-(((4-(1-methyl-4-(trifluoromethyl)-1*H*-imidazol-2-yl)bicyclo[2.2.2]octan-1-yl)methyl)amino)-[2,5'-bipyrimidine]-5-yl)dimethylphosphine oxide;
(4'-cyclopropyl-6'-methoxy-4-((((1*s*,2*R*,3*s*,4*r*,5*S*,6*r*,7*R*,8*S*)-4-(1-methyl-4-(trifluoromethyl)-1*H*-imidazol-2-yl)cuban-1-yl)methyl)amino)-[2,5'-bipyrimidine]-5-yl)dimethylphosphine oxide;
(4'-cyclopropyl-4-((4-(1-isopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-4-((3-fluoro-4-(1-isopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-4-((3-fluoro-4-(5-methyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((3-fluoro-4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-cyclopropyl-3-(trifluoromethyl)-1*H*-pyrazole-1-yl)benzyl)amino)-6'-methoxy-[2,5'-bipyrimidine]-5-yl)dimethylphosphine oxide; and
(4'-cyclopropyl-4-((4-(5-cyclopropyl-3-(trifluoromethyl)-1*H*-pyrazole-1-yl)-3-fluorobenzyl)amino)-6'-methoxy[2,5'-bipyrimidine]-5-yl)dimethylphosphine oxide.

The compound of the present invention may inhibit the activity of USP1.

Synthesis of the compound of the present invention

Compounds of the present invention, including Chemical Formula 1, may be synthesized by synthetic routes well known in the chemical arts, including those described herein and similar methods. Starting materials are generally available from commercial sources, such as Aldrich Chemicals (Milwaukee, Wisconsin), or are readily prepared using methods well known to those skilled in the art. For example, it can be prepared by methods generally described in the literature [Louis F. Fieser and Mary Fieser, Reagents for Organic Synthesis, 1st to 19th editions, Wiley, N.Y. (1967-1999)] or Beilsteins Handbuch der organischen Chemie, 4, Aufl (Springer-Verlag, Berlin, Germany).

For illustrative purposes, general methods for preparing the compounds and key intermediates of the present invention are outlined in the following reaction schemes. For more detailed descriptions of individual reaction steps, see the specific descriptions in the Examples below. Those skilled in the art will recognize that other synthetic routes can also be used to synthesize the compounds of the present invention. While specific starting materials and reagents are depicted in the reaction schemes and discussed below, other starting materials and reagents can be readily substituted to provide a variety of derivatives and/or reaction conditions. Furthermore, most compounds prepared by the methods described below can be further modified in light of the present disclosure using conventional chemistry well known to those skilled in the art.

The compound of Chemical Formula 1 according to the present invention may be prepared by a method represented by the following reaction scheme:

[Reaction scheme 1] Preparation method of the compound of Chemical Formula 1

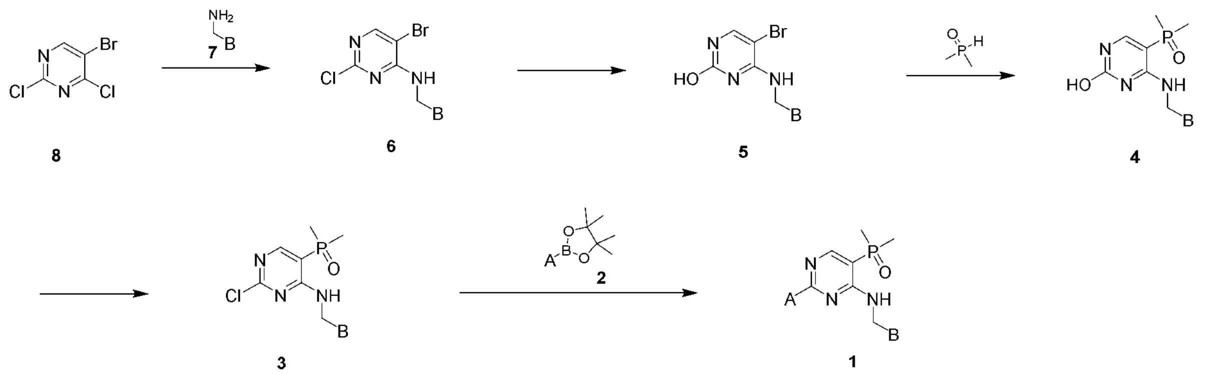

8 6 5 4
3 1

Compound 8 as starting material are reacted with and amine of Compound 7 to produce intermediate Compound 6. Subsequently, Compound 6 is prepared under acid conditions and reacted with dimethylphosphine oxide in the presence of a palladium catalyst to produce intermediate Compound 4. Subsequently, intermediate Compound 3 is prepared in the presence of phosphoryl chloride, and finally, is reacted with a boron derivative of Compound 2 to produce compound 1.

The compounds of the present invention may be used in a form enriched with deuterium as a substituent or functional group. Such deuterated forms may be obtained using the methods described in U.S. Patent Nos. 5,846,514 and 6,334,997, and reference may also be made to Dean, Dennis C., eds., <Recent Advances in the Synthesis and Applications of Radiolabeled Compounds for Drug Discovery and Development>, published in Curr., Pharm. Des.,

2000; 6 (10). Deuterium-enriched starting materials are commercially available, and deuterium-substituted functional groups may be obtained by applying such starting materials to the synthetic methods described herein.

Pharmaceutical Composition

In another aspect, the present invention discloses a pharmaceutical composition comprising the aforementioned compound and a pharmaceutically acceptable excipient. The pharmaceutical composition of the present invention may be used to inhibit enzyme activity of USP1 in the body, for example, for the treatment of cancer. In one embodiment, the pharmaceutical composition of the present invention may be used for the treatment of breast cancer. In a more specific embodiment, the pharmaceutical composition of the present invention may be used for the treatment of cancer, including BRCA-mutant cancer.

In the pharmaceutical composition of the present invention, the aforementioned compounds may be present as pharmaceutically acceptable salts. Pharmaceutically acceptable salts include, for example, base addition salts and acid addition salts. Pharmaceutically acceptable base addition salts may be formed with metals or amines, such as alkali and alkaline earth metals or organic amines. Pharmaceutically acceptable salts of the compounds may also be prepared using pharmaceutically acceptable cations. Pharmaceutically acceptable acid addition salts include inorganic or organic acid salts. Examples of acid addition salts include those derived from hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, benzoic acid, hydroxybenzoic acid, phenylacetic acid, cinnamic acid, salicylic acid, methanesulfonic acid, ethanesulfonic acid, benzenesulfonic acid, or toluenesulfonic acid.

As used herein, a pharmaceutically acceptable excipient refers to an inactive ingredient approved by a relevant administrative agency (e.g., the Ministry of Food and Drug Safety of the Republic of Korea, the Federal Food and Drug Administration (FDA) of the United States) as being suitable for use together with an active pharmaceutical ingredient for the purpose of manufacturing a medicine for treating a disease in humans or livestock. Such pharmaceutically acceptable excipients include, but are not limited to, carriers, lubricants, fluidizing agents, disintegrating agents, sweeteners, diluents, preservatives, coloring agents, flavoring agents, surfactants, wetting agents, dispersing agents, suspending agents, stabilizers, isotonic agents, solvent emulsifiers, and adjuvants.

The pharmaceutical composition of the present invention may be in a form suitable for oral administration, such as a tablet, capsule, pill, powder, sustained-release preparation, solution, or suspension; in a form suitable for parenteral injection, such as a sterile solution, suspension, or emulsion; in a form suitable for topical administration, such as an ointment or cream; or in a form suitable for rectal administration, such as a suppository.

The pharmaceutical composition according to the present invention may be prepared in a conventional manner, for example, by conventional mixing, dissolving, granulating, sugar-coated tablet preparation, powdering, emulsifying, encapsulating, enclosing, or lyophilizing processes. The appropriate formulation will vary depending on the chosen route of administration.

Pharmaceutical compositions suitable for oral administration may be readily formulated by combining the compounds disclosed herein with pharmaceutically acceptable excipients, such as carriers well known in the art. Using such excipients and carriers, the compounds disclosed herein may be formulated as tablets, pills, sugar-coated tablets, capsules, solutions, gels, syrups, slurries, suspensions, and the like for oral ingestion by patients to be treated. Oral pharmaceutical preparations may be obtained by adding the compounds of the present invention together with solid excipients, grinding the resulting mixture if necessary, adding suitable auxiliaries if necessary, and then processing the granulated mixture to form cores of tablets or sugar-coated tablets. Suitable excipients include, for example, fillers and cellulose preparations. **If** desired, a disintegrant may be added.

For pharmaceutical compositions for oral administration of a therapeutically effective amount of the compound of the present disclosure, the compositions are generally in the form of solid (e.g., tablets, capsules, pills, powders, or troches) or liquid formulations (e.g., aqueous suspensions, solutions, elixirs, or syrups).

When administered in tablet form, the composition may additionally contain functional solids and/or solid carriers such as gelatin or adjuvants. Compositions in the form of tablets, capsules, and powders may contain from about 1 to about 95 wt% of the compound of the present invention, preferably from about 15 to about 90 wt% of the compound of the present invention, based on the total weight of the composition. An example of a tablet composition may contain, for example, up to about 80 wt% of the active pharmaceutical ingredient, from about 10 to about 90 wt% of a binder, from about 0 to about 85 wt% of a diluent, from about 2 to about 10 wt% of a disintegrant, and from about 0.25 to about 10 wt% of a lubricant.

When administered in liquid or suspension form, a functional liquid and/or liquid carrier such as water, petroleum, or animal or vegetable oil may be added. Liquid compositions may further contain saline, sugar alcohol solutions, dextrose or other sugar solutions, or glycols. When administered in liquid or suspension form, the composition may contain from about 0.5 to about 90% by weight of the compound of the present invention, preferably from about 1 to about 50% by weight of the compound of the present invention. In one contemplated embodiment, the liquid carrier is non-aqueous or

substantially non-aqueous. For administration in liquid form, the composition may be supplied as a rapidly dissolving solid formulation for dissolution or suspension immediately prior to administration.

When the pharmaceutical composition of the present invention is administered intravenously, transdermally, or subcutaneously, it is in the form of a parenteral aqueous solution that does not contain a pyrogen. The preparation of such parenteral solutions by considering pH, isotonicity, stability, and the like, is within the skill of the art. Preferred compositions for intravenous, transdermal, or subcutaneous injection generally contain an isotonic vehicle. Such compositions may be prepared for administration as a solution of the free base or a pharmacologically acceptable salt in water suitably mixed with a surfactant such as hydroxypropyl cellulose. Dispersions in glycerol, liquid polyethylene glycol, and mixtures thereof, as well as dispersions in oils, may also be prepared. Under normal storage and use conditions, such preparations may optionally contain a preservative to prevent the growth of microorganisms.

Injectable compositions may include sterile aqueous solutions, suspensions, or dispersions, and sterile powders for the extemporaneous preparation of sterile injectable solutions, suspensions, or dispersions. Sterile injectable solutions are prepared by incorporating the active compound in the required amount in an appropriate solvent with various other ingredients, as required, followed by filtered sterilization. Generally, dispersions are prepared by incorporating the various sterile active ingredients into a sterile vehicle that contains a basic dispersion medium and the required other ingredients from those enumerated above. In embodiments of sterile powders for the preparation of sterile injectable solutions, the preferred methods of preparation are vacuum drying and freeze-drying techniques, which produce a powder of the active ingredient and any additional required ingredients from a previously sterile-filtered solution thereof.

To achieve controlled release of the active compound upon contact with body fluids in the gastrointestinal tract and to provide substantially constant and effective levels of the active compound in the plasma, sustained-release or sustained-release formulations may be prepared. For example, release may be controlled by one or more of dissolution, diffusion, and ion exchange. Furthermore, sustained-release approaches may enhance absorption through saturation or restriction pathways within the gastrointestinal tract. For example, for this purpose, the compound may be embedded in a polymer matrix comprising a biodegradable polymer, a water-soluble polymer, or a mixture thereof, and optionally a suitable surfactant. Embedding in this context may mean incorporating microparticles into the polymer matrix. Controlled-release formulations may also be obtained by encapsulating dispersed microparticles or emulsified microdroplets using known dispersion or emulsion coating techniques.

The pharmaceutical composition of the present invention may be formulated for parenteral administration by injection (e.g., bolus injection or continuous infusion). The injectable formulation may be presented in unit dosage form (e.g., ampoules or multi-dose containers) with an added preservative. The composition may take the form of a suspension, solution, or emulsion in an oily or aqueous vehicle, and may contain formulating agents such as suspending, stabilizing, and/or dispersing agents.

Pharmaceutical compositions may be presented in unit dosage forms suitable for single-dose administration of precise dosages.

Pharmaceutical compositions comprising the compounds of the present invention may be used according to the methods described below.

## Method of Treatment

In another aspect, the present invention provides a method of treating a disease requiring inhibition of USP1 activity. This method comprises administering a therapeutically effective amount of a compound of the present invention, such as a compound defined by Chemical Formula 1, or a pharmaceutical composition comprising such a compound of the present invention, to a patient requiring inhibition of USP1 activity.

Diseases requiring inhibition of USP1 activity include cancer, diabetes, and metabolic diseases, including obesity.

In one embodiment, the present invention provides a method for treating cancer. This method comprises administering to a cancer patient a therapeutically effective amount of a compound of the present invention, such as a compound defined by Chemical Formula 1, or a pharmaceutical composition comprising the compound of the present invention. In one specific embodiment, the cancer is a BRCA-mutant cancer.

In the method for treating cancer, the compound of the present invention may be administered alone or in combination with at least one other drug. These other drugs and the compound of the present invention may be administered simultaneously or sequentially. Examples of drugs that may be administered in combination with the drug of the present invention include PARP inhibitors and cytotoxic anticancer agents.

As used herein, unless otherwise specified, a "therapeutically effective amount" of a compound refers to an amount of the compound sufficient to delay or minimize one or more symptoms associated with a disease, disorder, or condition, or to provide a therapeutic effect against a disease, disorder, or condition. The term "therapeutically effective amount" encompasses an amount that improves overall healing, an amount that alleviates or avoids symptoms or causes of the disease or condition, and an amount that enhances the therapeutic efficacy of other treatments.

The amount of the compound administered may vary depending on the subject being treated, the subject's age, health, sex, and weight, the type of concurrent treatment (if any), the severity of the pain, the nature of the desired effect, the mode and frequency of treatment, and the judgment of the prescribing physician. The frequency of administration may also vary depending on the pharmacodynamic effects on arterial oxygen pressure. However, the most desirable dosage can be tailored to the individual subject, as understood and determined by one of skill in the art without undue experimentation. This usually involves adjusting the standard dose (e.g., reducing the dose if the patient is underweight).

Although individual case needs may vary, determining the optimal range of therapeutically effective doses of a compound is within the skill of the art. When administered to humans in the therapeutic or prophylactic treatment of conditions and disorders using the compounds of the present invention, for example, a typical dosage of the compounds of the present invention may be from about 0.05 mg/kg/day to about 50 mg/kg/day, for example, at least 0.05 mg/kg, at least 0.08 mg/kg, at least 0.1 mg/kg, at least 0.2 mg/kg, at least 0.3 mg/kg, at least 0.4 mg/kg, or at least 0.5 mg/kg, preferably not more than 50 mg/kg, not more than 40 mg/kg, not more than 30 mg/kg, not more than 20 mg/kg, or not more than 10 mg/kg, which may be, for example, from about 2.5 mg/day (0.5 mg/kg x 5 kg) to about 5000 mg/day (50 mg/kg x 100 kg). For example, the dosage of the compound may be about 0.1 mg/kg/day to about 50 mg/kg/day, about 0.05 mg/kg/day to about 10 mg/kg/day, about 0.05 mg/kg/day to about 5 mg/kg/day, about 0.05 mg/kg/day to about 3 mg/kg/day, about 0.07 mg/kg/day to about 3 mg/kg/day, about 0.09 mg/kg/day to about 3 mg/kg/day, about 0.05 mg/kg/day to about 0.1 mg/kg/day, about 0.1 mg/kg/day to about 1 mg/kg/day, about 1 mg/kg/day to about 10 mg/kg/day, about 1 mg/kg/day to about 5 mg/kg/day, about 1 mg/kg/day to about 3 mg/kg/day, about 3 mg/day to about 500 mg/day, about 5 mg/day to about 250 mg/day, about 10 mg/day to about 100 mg/day, about 3 mg/day to about 10 mg/day, or about 100 mg/day to about 250 mg/day. These doses may be administered as a single dose or divided into multiple doses.

In another aspect of the present invention, a method of inhibiting intracellular activity of USP1 is provided. The method comprises contacting a cell in which inhibition of USP1 activity is desired, with an effective amount of a compound of the present invention - e.g., a compound defined by Chemical Formula 1.

## [EXAMPLES]

Hereinafter, the present invention will be described in more detail through the following examples and experimental examples. However, these examples and experimental examples are intended only to aid understanding of the present invention and are not intended to limit the scope of the present invention in any way. Various changes and modifications may be made to the present examples, and such changes and modifications also fall within the scope of the appended claims.

The full names of the abbreviations used in the synthetic methods below are as follows:

Ph: Phenyl
TEA: Triethylamine
THF: Tetrahydrofuran
$Pd_2(dba)_3$: Tris(dibenzylideneacetone)dipalladium
XantPhos: (9,9-dimethyl-9*H*-xanthene-4,5-diyl)bis(diphenylphosphane)

[Example 1]

**Preparation of (2-(2-cyclopropyl-6-methoxyphenyl)-4-((4-(1-methyl-4-(trifluoromethyl)-1*H*-imidazol-2-yl)benzyl) amino)pyrimidin-5-yl)dimethylphosphine oxide**

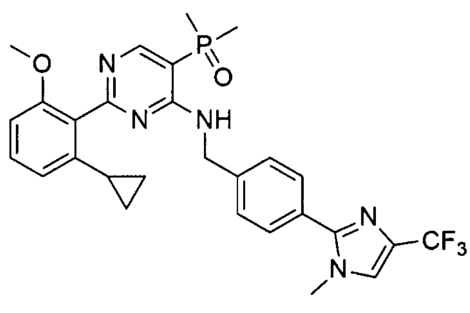

Compound of Example 1 was synthesized according to the following reaction scheme.

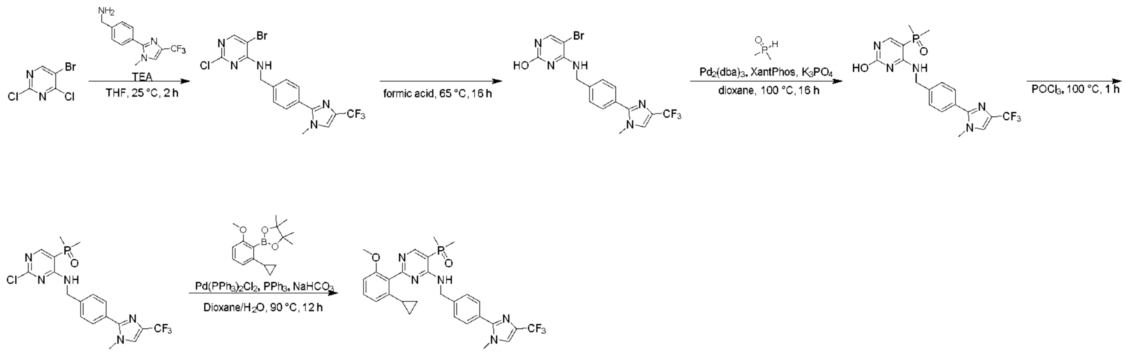


Step 1) Preparation of 5-bromo-2-chloro-*N*-(4-(1-methyl-4-(trifluoromethyl)-1*H*-imidazol-2-yl)benzyl)pyrimidin-4-amine

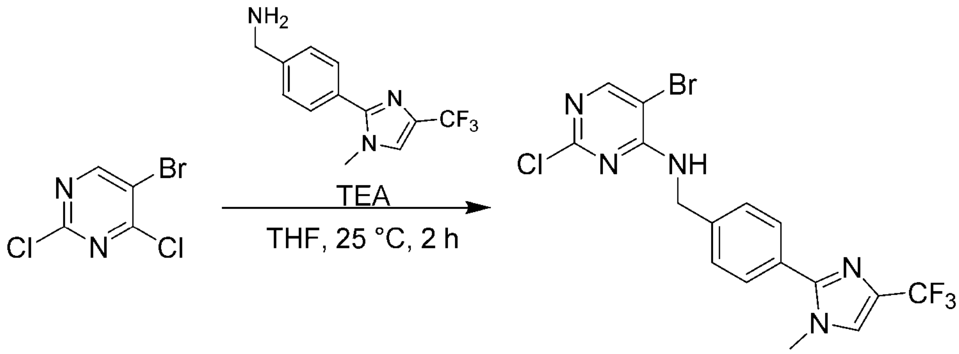


(4-(1-methyl-4-(trifluoromethyl)-1H-imidazol-2-yl)phenyl)methanamine (2.20 g, 8.62 mmol, 1.00 equivalent) was dissolved in tetrahydrofuran (20.0 mL), and 5-bromo-2,4-dichloropyrimidine (1.96 g, 8.62 mmol, 1.10 mL, 1.00 equivalent) and triethylamine (1.74 g, 17.2 mmol, 2.40 mL, 2.00 equivalent) were added, and the mixture was stirred at 25 °C for 2 hours. The reaction mixture was dried under reduced pressure to obtain a residue. The residue was purified by reverse phase chromatography ($C_{18}$, 330 g; conditions: water/acetonitrile = 100:0 to 0:100, 0.1% formic acid) and concentrated under reduced pressure to obtain 5-bromo-2-chloro-*N*-(4-(1-methyl-4-(trifluoromethyl)-1*H*-imidazol-2-yl)benzyl)pyrimidin-4-amine (2.60 g, 5.82 mmol, 67.5% yield) as a brown solid.

$^1$H NMR (400 MHz, DMSO-$d_6$) $\delta$ = 8.42 (br t, *J* = 6.0 Hz, 1H), 8.30 (s, 1H), 7.92 (s, 1H), 7.69 (d, *J* = 8.0 Hz, 2H), 7.44 (d, *J* = 8.0 Hz, 2H), 4.65 (br d, *J* = 6.0 Hz, 2H), 3.77 (s, 3H).

Step 2) Preparation of 5-bromo-4-((4-(1-methyl-4-(trifluoromethyl)-1*H*-imidazol-2-yl)benzyl)amino)pyrimidin-2-ol

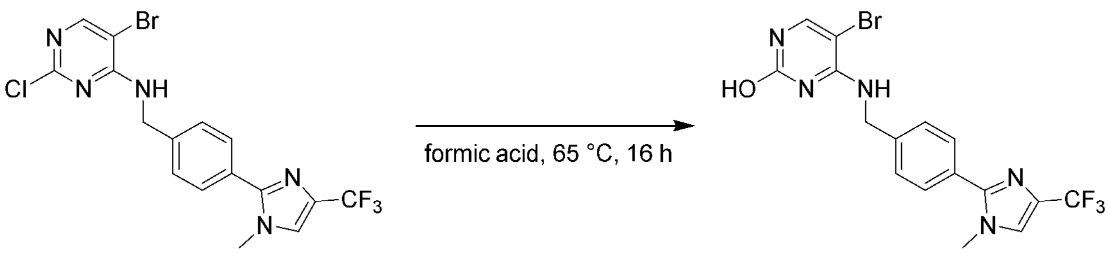


A solution containing 5-bromo-2-chloro-*N*-(4-(1-methyl-4-(trifluoromethyl)-1*H*-imidazol-2-yl)benzyl)pyrimidin-4-amine (2.60 g, 5.82 mmol, 1.00 equiv) in formic acid (26.0 mL) was stirred at 65 °C for 16 h. The reaction mixture was dried under reduced pressure to obtain a residue. Subsequently, The residue was performed by tritylation in methanol (3 mL) at 20 °C for 1 h, followed by filtration to obtain 5-bromo-4-((4-(1-methyl-4-(trifluoromethyl)-1*H*-imidazol-2-yl)benzyl)amino)pyrimidin-2-ol (2.40 g, 5.60 mmol, 96.3% yield) as a white solid.

$^1$H NMR (400 MHz, DMSO-$d_6$) $\delta$ = 7.96 - 7.91 (m, 2H), 7.78 (s, 1H), 7.67 (d, *J* = 8.0 Hz, 2H), 7.40 (d, *J* = 8.0 Hz, 2H),

4.61 (d, $J$ = 6.0 Hz, 2H), 3.77 (s, 3H).

Step 3) Preparation of (2-hydroxyl-4-((4-(1-methyl-4-(trifluoromethyl)-1*H*-imidazol-2-yl)benzyl)amino)pyrimidin-5-yl)dimethylphosphine oxide

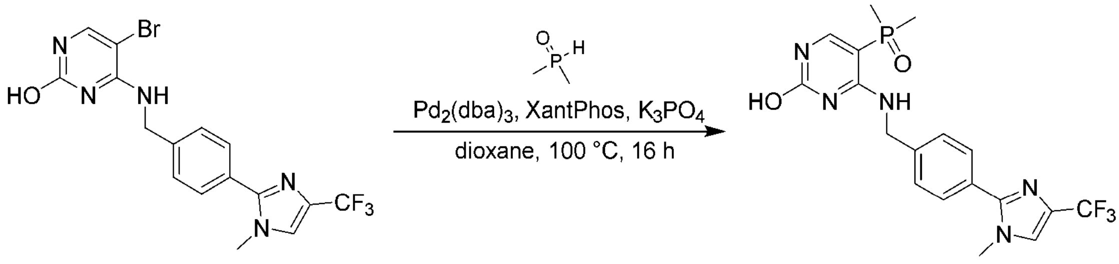


To a solution containing 5-bromo-4-((4-(1-methyl-4-(trifluoromethyl)-1*H*-imidazol-2-yl)benzyl)amino)pyrimidin-2-ol (1.00 g, 2.34 mmol, 1.00 equiv) was added with dioxane (10.0 mL), dimethylphosphine oxide (364 mg, 4.67 mmol, 2.00 equiv), potassium phosphate (545 mg, 2.57 mmol, 1.10 equiv), tris(dibenzylideneacetone)dipalladium(0) (64.2 mg, 70.1 μmol, 0.0300 equiv), and 4,5-bis(diphenylphosphino)-9,9-dimethylxanthene (81.1 mg, 140 μmol, 0.0600 equiv). The mixture was treated three times by vacuum and nitrogen flushing, and stirred at 100 °C for 16 h under a nitrogen atmosphere. The reaction mixture was filtered, and the filtrate was dried under reduced pressure to obtain a residue. The residue was tritylated in methanol (2.00 mL) at 20 °C for 1 h, filtered, and the filtrate was concentrated under reduced pressure to obtain (2-hydroxy-4-((4-(1-methyl-4-(trifluoromethyl)-1*H*-imidazol-2-yl)benzyl)amino)pyrimidin-5-yl)dimethylphosphine oxide (700 mg, 1.65 mmol, 70.5% yield) as a yellow solid.

$^1$H NMR (400 MHz, DMSO-$d_6$) δ = 11.49 - 10.85 (m, 1H), 8.95 (t, $J$ = 6.0 Hz, 1H), 7.93 (d, $J$ = 0.8 Hz, 1H), 7.82 (d, $J$ = 10.4 Hz, 1H), 7.69 (d, $J$ = 8.4 Hz, 2H), 7.43 (d, $J$ = 8.4 Hz, 2H), 4.64 (d, $J$ = 5.6 Hz, 2H), 3.78 (s, 3H), 1.71 (d, $J$ = 13.6 Hz, 6H).

Step 4) Preparation of (2-chloro-4-((4-(1-methyl-4-(trifluoromethyl)-1*H*-imidazol-2-yl)benzyl)amino)pyrimidin-5-yl)dimethylphosphine oxide

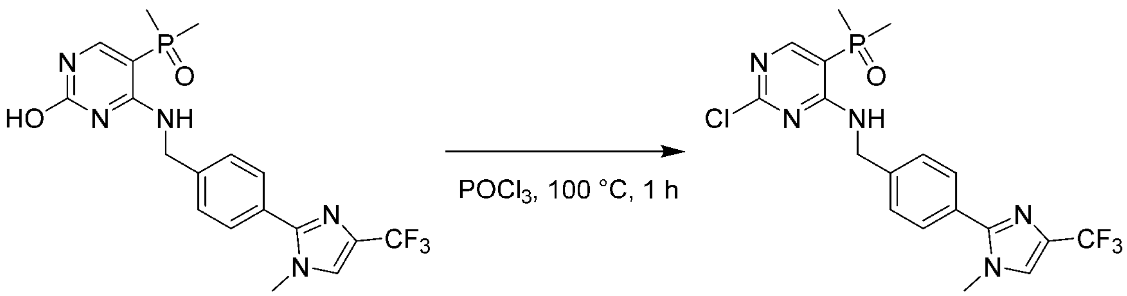


A solution containing (2-hydroxy-4-((4-(1-methyl-4-(trifluoromethyl)-1*H*-imidazol-2-yl)benzyl)amino)pyrimidin-5-yl)dimethylphosphine oxide (680 mg, 1.60 mmol, 1.00 equiv) was stirred with phosphoryl chloride (7.00 mL) at 100 °C for 1 h. The reaction mixture was dried under reduced pressure to obtain a residue. The residue was purified by Prep-HPLC (Column: Phenomenex Luna C18 150 x 40 mm x 15 μm; Mobile phase: [water (formic acid) - acetonitrile]; Gradient: 30%-60% B for 15 min) and dried to obtain (2-chloro-4-((4-(1-methyl-4-(trifluoromethyl)-1*H*-imidazol-2-yl)benzyl)amino)pyrimidin-5-yl)dimethylphosphine oxide (180 mg, 405 μmol, 25.4% yield) as a white solid.

$^1$H NMR (400 MHz, DMSO-$d_6$) δ = 9.20 (t, $J$ = 5.6 Hz, 1H), 8.30 (d, $J$ = 8.4 Hz, 1H), 7.93 (d, $J$ = 0.8 Hz, 1H), 7.71 (d, $J$ = 8.0 Hz, 2H), 7.47 (d, $J$ = 8.4 Hz, 2H), 4.68 (d, $J$ = 5.6 Hz, 2H), 3.78 (s, 3H), 1.80 (d, $J$ = 14 Hz, 6H).

Step 5) Preparation of (2-(2-cyclopropyl-6-methoxyphenyl)-4-((4-(1-methyl-4-(trifluoromethyl)-1*H*-imidazol-2-yl)benzyl)amino)pyrimidin-5-yl)dimethylphosphine oxide

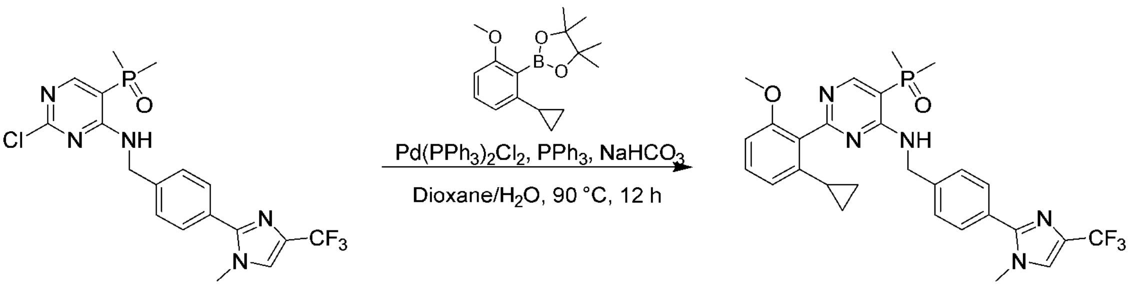


(2-Hydroxy-4-((4-(1-methyl-4-(trifluoromethyl)-1*H*-imidazol-2-yl)benzyl)amino)pyrimidin-5-yl)dimethylphosphine oxide (80.0 mg, 180 μmol, 1.00 equiv), 2-(2-cyclopropyl-6-methoxyphenyl)-4,4,5,5-tetramethyl-1,3,2-dioxoborolane (98.8 mg, 360 μmol, 2.00 equiv), sodium bicarbonate (45.4 mg, 541 μmol, 21.0 μL, 3.00 equiv), triphenylphosphine (2.36 mg, 9.01 μmol, 0.0500 equiv), and bis(triphenylphosphine)palladium(II) chloride (6.33 mg, 9.01 μmol, 0.0500 (equivalent) was added to dioxane (1.00 mL) and water (0.200 mL), and the mixture was stirred at 90°C for 12 h in a nitrogen atmosphere after three vacuum and nitrogen flushing treatments. The reaction mixture was filtered, and the filtrate was dried under reduced pressure to obtain a residue. The residue was purified by Prep-HPLC (Column: Waters Xbridge 150 × 25 mm × 5 μm; Mobile phase: [water (ammonium hydroxide) - acetonitrile]; Gradient: 30%-60% B for 10 min) and dried to obtain (2-(2-cyclopropyl-6-methoxyphenyl)-4-((4-(1-methyl-4-(trifluoromethyl)-1*H*-imidazol-2-yl)benzyl)amino)pyrimidin-5-yl)dimethylphosphine oxide (9.05 mg, 15.70 μmol, 8.71% yield, 96.4% purity) as a yellow solid.

$^1H$ NMR (400 MHz, DMSO-$d_6$) δ = 8.82 (t, *J* = 5.6 Hz, 1H), 8.47 (d, *J* = 8.8 Hz, 1H), 7.92 (s, 1H), 7.65 (d, *J* = 8.0 Hz, 2H), 7.42 (d, *J* = 8.0 Hz, 2H), 7.23 (t, *J* = 8.0 Hz, 1H), 6.86 (d, *J* = 8.4 Hz, 1H), 6.49 (d, *J* = 7.6 Hz, 1H), 4.69 (br d, *J* = 5.6 Hz, 2H), 3.76 (s, 3H), 3.64 (s, 3H), 1.84 (d, *J* = 13.6 Hz, 6H), 1.47 - 1.35 (m, 1H), 0.67 - 0.57 (m, 2H), 0.54 - 0.44 (m, 2H).

[Example 2]

**Preparation of (4'-cyclopropyl-6'-methoxy-4-((4-(1-methyl-4-(trifluoromethyl)-1*H*-imidazol-2-yl)benzyl)amino)-[2,5'-bipyrimidine]-5-yl)dimethylphosphine oxide**

The titled compound was prepared by a method similar to the preparation method of Example 1, starting from the starting material (2-chloro-4-((4-(1-methyl-4-(trifluoromethyl)-1*H*-imidazol-2-yl)benzyl)amino)pyrimidin-5-yl)dimethylphosphine oxide according to the following reaction scheme.

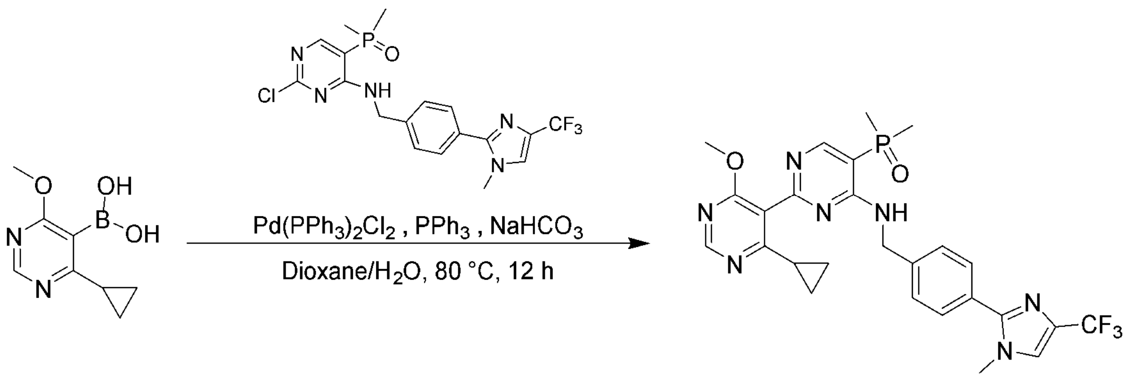


$^1H$ NMR (400 MHz, DMSO-$d_6$) δ = 8.96 (br t, *J* = 5.6 Hz, 1H), 8.62 (s, 1H), 8.51 (d, *J* = 8.4 Hz, 1H), 7.92 (d, *J* = 0.8 Hz, 1H), 7.67 (d, *J* = 8.0 Hz, 2H), 7.44 (d, *J* = 8.0 Hz, 2H), 4.70 (br d, *J* = 6.0 Hz, 2H), 3.83 (s, 3H), 3.76 (s, 3H), 1.86 (s, 3H), 1.83 (s, 3H), 1.77 - 1.71 (m, 1H), 1.02 - 0.96 (m, 2H), 0.86 - 0.78 (m, 2H).

[Example 3]

**Preparation of (2-(1-isopropyl-4-methyl-1*H*-pyrazole-5-yl)-4-((4-(1-methyl-4-(trifluoromethyl)-1*H*-imidazol-2-yl) benzyl)amino)pyrimidin-5-yl)dimethylphosphine oxide**

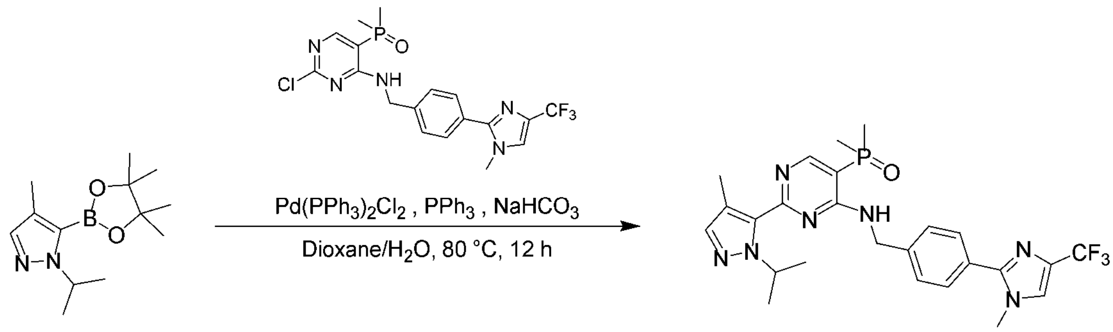


1-Isopropyl-4-methyl-5-(4,4,5,5-tetramethyl-1,3,2-dioxaborolan-2-yl)-1*H*-pyrazole (338 mg, 676 μmol, 1.50 equiv) was dissolved in dioxane (4.00 mL) and water (1.00 mL), 2-chloro-5-dimethylphosphoryl-*N*-[[4-[1-methyl-4-(trifluoromethyl)imidazol-2-yl]phenyl]methyl]pyrimidin-4-amine (200 mg, 450 μmol, 1.00 equiv), bis(triphenylphosphine) palladium(II) chloride (15.8 mg, 22.5 μmol, 0.0500 equiv), triphenylphosphine (5.91 mg, 22.5 μmol, 0.0500 equiv), saturated Sodium carbonate (113 mg, 1.35 mmol, 52.6 μL, 3.00 equiv) was added. The mixture was stirred at 80 °C for 12 h under a nitrogen atmosphere. The reaction mixture was concentrated under reduced pressure to obtain a residue, which was purified by Prep-HPLC (column: Phenomenex Luna C18 150 x 25 mm x 10 μm; mobile phase: [water (formic acid) - acetonitrile]; gradient: 38%-68% B for 9 min) and dried to obtain (2-(1-isopropyl-4-methyl-1H-pyrazole-5-yl)-4-((4-(1-methyl-4-(trifluoromethyl)-1*H*-imidazol-2-yl)benzyl)amino)pyrimidin-5-yl)dimethylphosphine oxide (6.42 mg, 11.35 μmol, 2% yield, 94% purity) as a gray solid.

$^1$H NMR (400 MHz, DMSO-$d_6$) δ = 8.97 (t, *J* = 6.0 Hz, 1H), 8.55 (d, *J* = 8.4 Hz, 1H), 7.92 (d, *J* = 0.8 Hz, 1H), 7.70 (d, *J* = 8.4 Hz, 2H), 7.44 (d, *J* = 8.4 Hz, 2H), 7.33 (s, 1H), 5.50 - 5.35 (m, 1H), 4.79 (d, *J* = 6.0 Hz, 2H), 3.76 (s, 3H), 2.13 (s, 3H), 1.86 (s, 3H), 1.83 (s, 3H), 1.26 (s, 3H), 1.24 (s, 3H).

[Example 4]

**Preparation of (2-(2-isopropylpyridine-3-yl)-4-((4-(1-methyl-4-(trifluoromethyl)-1*H*-imidazol-2-yl)benzyl)amino) pyrimidin-5-yl)dimethylphosphine oxide**

The titled compound was prepared according to the following reaction scheme.

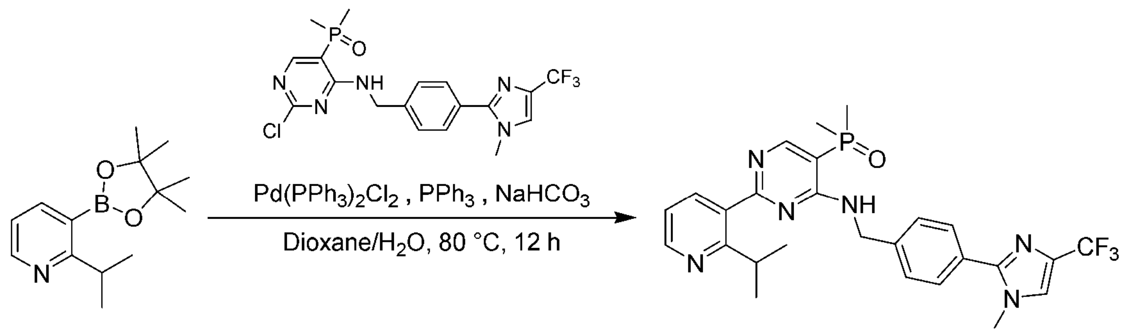


(2-Chloro-4-((4-(1-methyl-4-(trifluoromethyl)-1H-imidazol-2-yl)benzyl)amino)pyrimidin-5-yl)dimethylphosphine oxide (100 mg, 225 μmol, 1.00 equiv) was dissolved in dioxane (2.00 mL), 2-isopropyl-3-(4,4,5,5-tetramethyl-1,3,2-dioxaborolan-2-yl)pyridine (123 mg, 496 μmol, 2.20 equiv), sodium carbonate (56.8 mg, 676 μmol, 26.3 μL, 3.00 equiv), triphenylphosphine (2.96 mg, 11.3 μmol, 0.0500 equiv), bis(triphenylphosphine)palladium(II) chloride (7.91 mg, 11.3 μmol, 0.0500 equivalents) was added. The mixture was stirred at 80 °C for 12 hours under a nitrogen atmosphere. The reaction mixture was concentrated under reduced pressure to obtain a residue, which was purified by column chromatography ($SiO_2$, petroleum ether/ethyl acetate/methanol = 1/0/0 to 0/20/1). Subsequently, after further purification by Prep-HPLC (column: YMC-Actus Triart C18 150 x 30 mm x 7 μm; mobile phase: [water (formic acid) - acetonitrile]; gradient: 20%-50% B for 10 min), the residue was freeze-dried to obtain (2-(2-isopropylpyridine-3-yl)-4-((4-(1-methyl-4-(trifluoromethyl)-1*H*-imidazol-2-yl)benzyl)amino)pyrimidin-5-yl)dimethylphosphine oxide (17.04 mg, 32.24 μmol, 14% yield) as a white solid.

$^1$H NMR (400 MHz, DMSO-$d_6$) δ = 8.97 (t, *J* = 5.6 Hz, 1H), 8.59 (dd, *J* = 1.6, 4.8 Hz, 1H), 8.55 (d, *J* = 8.4 Hz, 1H), 7.96 - 7.90 (m, 2H), 7.69 (d, *J* = 8.4 Hz, 2H), 7.44 (d, *J* = 8.4 Hz, 2H), 7.28 (dd, *J* = 4.8, 7.6 Hz, 1H), 4.78 (d, *J* = 6.0 Hz, 2H), 3.77 (s, 3H), 3.72 (td, *J* = 6.8, 13.2 Hz, 1H), 1.85 (d, *J* = 13.6 Hz, 6H), 1.10 (d, *J* = 6.8 Hz, 6H).

[Example 5]

**Preparation of (4'-cyclopropyl-6'-(methoxy-*d*$_3$)-4-((4-(1-methyl-4-(trifluoromethyl)-1*H*-imidazol-2-yl)benzyl)amino)-[2,5'-bipyrimidine]-5-yl)dimethylphosphine oxide**

The titled compound was prepared according to the following reaction scheme.

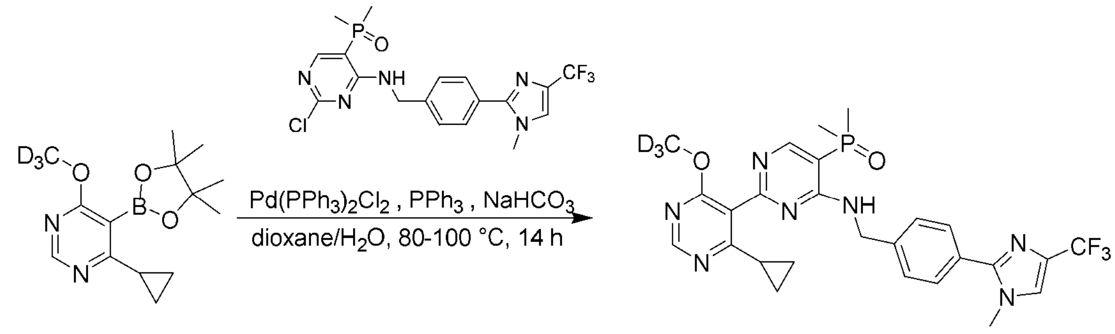


4-cyclopropyl-6-(methoxy-*d*$_3$)-5-(4,4,5,5-tetramethyl-1,3,2-dioxaborolan-2-yl)pyrimidine (151 mg, 541 μmol, 1.50 equiv) and (2-chloro-4-((4-(1-methyl-4-(trifluoromethyl)-1H-imidazol-2-yl)benzyl)amino)pyrimidin-5-yl)dimethylphosphine oxide (160 mg, 361 μmol, 1.00 equiv) were dissolved in dioxane (2.00 mL) and water (0.400 mL), and sodium carbonate (90.9 mg, 1.08 mmol, 42.1 μL, 3.00 equiv), triphenylphosphine (4.73 mg, 18.0 μmol, 0.0500 equiv), Bis(triphenylphosphine)palladium(II) chloride (12.7 mg, 18.0 μmol, 0.0500 eq) was added. The mixture was stirred at 80 °C for 12 h under a nitrogen atmosphere, then the mixture was heated to 100 °C and stirred for 2 h. The reaction mixture was concentrated under reduced pressure to obtain a residue, which was purified by column chromatography ($SiO_2$, dichloroethane/isopropyl alcohol=1/0 to 10/1), purified again by Prep-HPLC (column: Phenomenex Luna C18 250 x 50 mm x15 μm; mobile phase: [water (formic acid) - acetonitrile]; gradient: 28%-58% B for 9 min), and lyophilized to obtain (4'-cyclopropyl-6'-(methoxy-*d*$_3$)-4-((4-(1-methyl-4-(trifluoromethyl)-1*H*-imidazol-2-yl)benzyl)amino)-[2,5'-bipyrimidine]-5-yl) dimethylphosphine oxide (23.4 mg, 41.08 μmol, 11.4% yield) as a light gray solid. (98.4% purity) was obtained.

$^1$H NMR (400 MHz, DMSO-*d*$_6$) δ = 8.97 (t, *J* = 6.0 Hz, 1H), 8.62 (s, 1H), 8.51 (d, *J* = 8.4 Hz, 1H), 7.92 (s, 1H), 7.67 (d, *J* = 8.0 Hz, 2H), 7.44 (d, *J* = 8.0 Hz, 2H), 4.69 (d, *J* = 6.0 Hz, 2H), 3.77 (s, 3H), 1.86 (s, 3H), 1.83 (s, 3H), 1.78 - 1.69 (m, 1H), 0.99 (td, *J* = 3.6, 7.2 Hz, 2H), 0.86 - 0.78 (m, 2H).

[Example 6]

**Preparation of (2-(2-(difluoromethoxy)pyridin-3-yl)-4-((4-(1-methyl-4-(trifluoromethyl)-1*H*-imidazol-2-yl)benzyl) amino)pyrimidin-5-yl)dimethylphosphine oxide**

The titled compound was prepared by a method similar to the preparation method of Example 1, starting from the starting material (2-chloro-4-((4-(1-methyl-4-(trifluoromethyl)-1H-imidazol-2-yl)benzyl)amino)pyrimidin-5-yl)dimethylphosphine oxide according to the following reaction scheme.

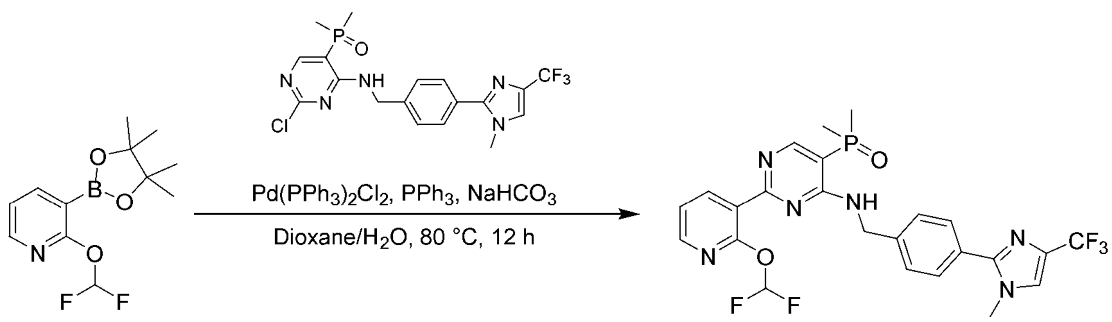


$^1$H NMR (400 MHz, DMSO-*d*$_6$) δ = 8.96 (br t, *J* = 6.0 Hz, 1H), 8.54 (d, *J* = 8.8 Hz, 1H), 8.36 (dd, *J* = 1.6, 4.8 Hz, 1H), 8.29 (dd, *J* = 1.6, 7.6 Hz, 1H), 7.91 (d, *J* = 0.8 Hz, 1H), 7.80 (t, *J* = 72.8, 1H), 7.68 (d, *J* = 8.0 Hz, 2H), 7.49 (d, *J* = 8.0 Hz, 2H), 7.40 (dd, *J* = 4.8, 7.6 Hz, 1H), 4.79 (d, *J* = 5.6 Hz, 2H), 3.76 (s, 3H), 1.83 (d, *J* = 13.6 Hz, 6H).

[Example 7]

**Preparation of (4'-cyclopropyl-4-((3-fluoro-4-(1-methyl-4-(trifluoromethyl)-1*H*-imidazol-2-yl)benzyl)amino)-6'-methoxy-[2,5'-bipyrimidine]-5-yl)dimethylphosphine oxide**

The titled compound was prepared by a method similar to the preparation method of Example 1, starting from the starting material (3-fluoro-4-(1-methyl-4-(trifluoromethyl)-1*H*-imidazol-2-yl)phenyl)methanamine according to the following reaction scheme.

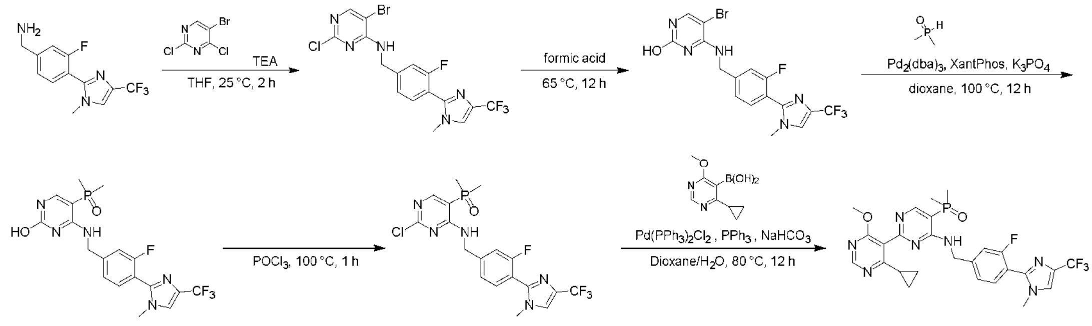


$^1$H NMR (400 MHz, DMSO-$d_6$) δ = 9.00 (t, *J* = 6.0 Hz, 1H), 8.62 (s, 1H), 8.52 (d, *J* = 8.4 Hz, 1H), 7.99 (d, *J* = 1.2 Hz, 1H), 7.54 (t, *J* = 7.6 Hz, 1H), 7.34 - 7.28 (m, 2H), 4.72 (d, *J* = 6.0 Hz, 2H), 3.82 (s, 3H), 3.59 (d, *J* = 1.2 Hz, 3H), 1.87 (s, 3H), 1.84 (s, 3H), 1.76 - 1.69 (m, 1H), 1.03 - 0.97 (m, 2H), 0.86 - 0.80 (m, 2H).

[Example 8]

**Preparation of (4'-cyclopropyl-4-((4-(1-cyclopropyl-4-(trifluoromethyl)-1*H*-imidazol-2-yl)benzyl)amino)-6'-methoxy-[2,5'-bipyrimidine]-5-yl)dimethylphosphine oxide**

The titled compound was prepared from the starting material (4-(1-cyclopropyl-4-(trifluoromethyl)-1*H*-imidazol-2-yl)phenyl)methanamine by a method similar to the preparation method of Example 1 according to the following reaction scheme.

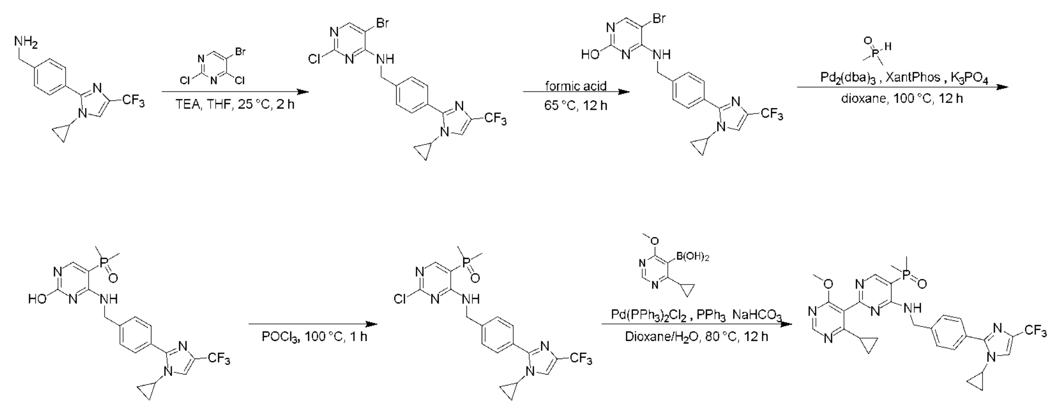


$^1$H NMR (400 MHz, DMSO-$d_6$) δ = 8.98 (t, *J* = 6.0 Hz, 1H), 8.62 (s, 1H), 8.51 (d, *J* = 8.4 Hz, 1H), 7.93 (d, *J* = 1.2 Hz, 1H), 7.84 (d, *J* = 8.4 Hz, 2H), 7.43 (d, *J* = 8.4 Hz, 2H), 4.70 (d, *J* = 6.0 Hz, 2H), 3.83 (s, 3H), 3.73 (tt, *J* = 3.6, 7.6 Hz, 1H), 1.85 (d, *J* = 13.6 Hz, 6H), 1.77 - 1.71 (m, 1H), 1.01 - 0.94 (m, 4H), 0.92 - 0.87 (m, 2H), 0.85 - 0.78 (m, 2H).

[Example 9]

**Preparation of (4'-cyclopropyl-6'-methoxy-4-((4-(5-methyl-3-(trifluoromethyl)-1*H*-pyrazole-1-yl)benzyl)amino)-[2,5'-bipyrimidine]-5-yl)dimethylphosphine oxide**

The titled compound was prepared from the starting material (4-(5-methyl-3-(trifluoromethyl)-1*H*-pyrazole-1-yl) phenyl)methanamine by a method similar to the preparation method of Example 1 according to the following reaction

scheme.

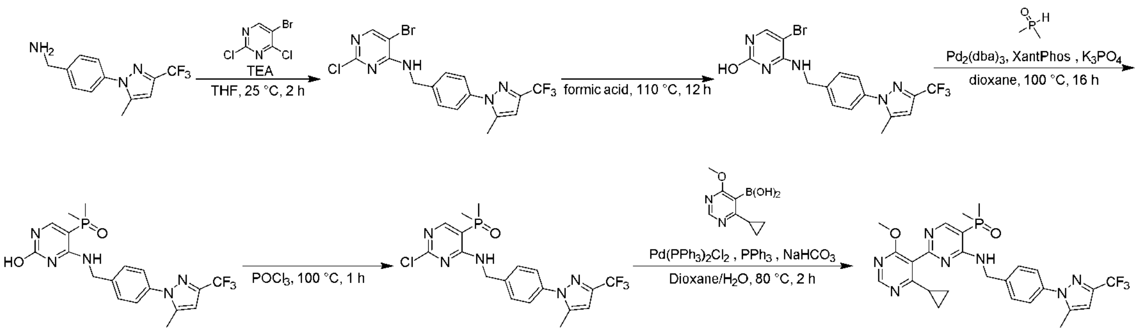


$^1$H NMR (400 MHz, DMSO-$d_6$) δ = 8.99 (t, *J* = 6.4 Hz, 1H), 8.62 (s, 1H), 8.51 (d, *J* = 8.4 Hz, 1H), 7.55 - 7.50 (m, 2H), 7.50 - 7.45 (m, 2H), 6.75 (s, 1H), 4.71 (d, *J* = 5.6 Hz, 2H), 3.83 (s, 3H), 2.32 (s, 3H), 1.87 (s, 3H), 1.83 (s, 3H), 1.77 - 1.67 (m, 1H), 1.02 - 0.94 (m, 2H), 0.86 - 0.76 (m, 2H).

[Example 10]

**Preparation of (4'-cyclopropyl-6'-methoxy-4-(((4-(1-methyl-4-(trifluoromethyl)-1*H*-imidazol-2-yl)bicyclo[2.2.2] octan-1-yl)methyl)amino)-[2,5'-bipyrimidine]-5-yl)dimethylphosphine oxide**

The titled compound was prepared from the starting material (4-(1-methyl-4-(trifluoromethyl)-1H-imidazol-2-yl) bicyclo[2.2.2]octan-1-yl)methanamine by a method similar to the preparation method of Example 1 according to the following reaction scheme.

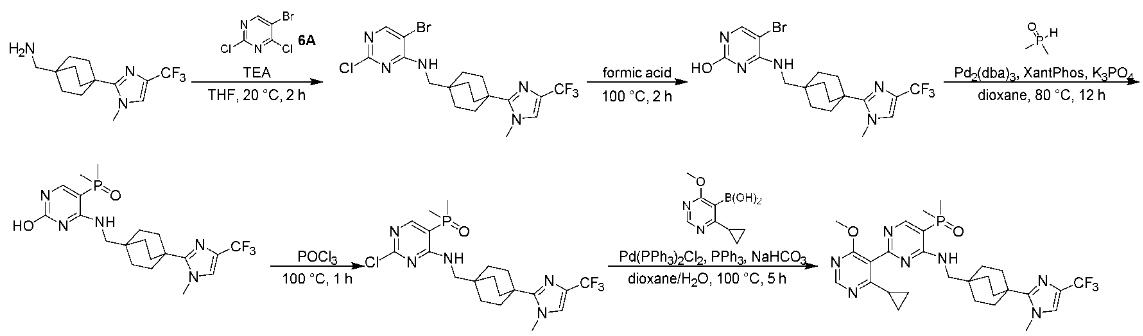


$^1$H NMR (400 MHz, DMSO-$d_6$) δ = 9.43 (br s, 1H), 8.72 (s, 1H), 8.51 (d, *J* = 9.2 Hz, 1H), 7.65 (s, 1H), 3.90 (s, 3H), 3.76 (s, 3H), 3.28 (br d, *J* = 6.0 Hz, 2H), 1.95 - 1.85 (m, 13H), 1.54 - 1.44 (m, 6H), 1.14 - 1.06 (m, 2H), 0.98 (br dd, *J* = 3.2, 7.6 Hz, 2H).

[Example 11]

**Preparation of (4'-cyclopropyl-6'-methoxy-4-((((1*s*,2*R*,3*s*,4*r*,5*S*,6*r*,7*R*,8*S*)-4-(1-methyl-4-(trifluoromethyl)-1*H*-imidazol-2-yl)cuban-1-yl)methyl)amino)-[2,5'-bipyrimidine]-5-yl)dimethylphosphine oxide**

The titled compound was prepared from the starting material ((1s,2R,3s,4r,5S,6r,7R,8S)-4-(1-methyl-4-(trifluoromethyl)-1H-imidazol-2-yl)cuban-1-yl)methanamine by a method similar to the preparation method of Example 1 below, according to the following reaction scheme.

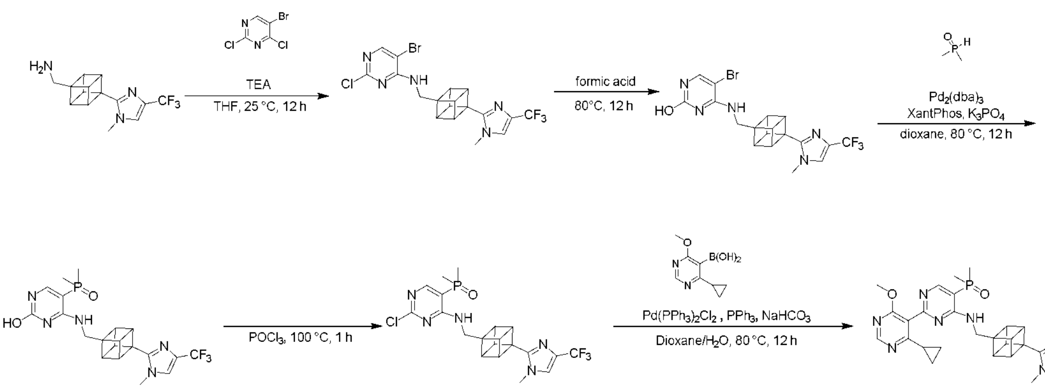


$^1H$ NMR (400 MHz, DMSO-$d_6$) $\delta$ = 8.68 - 8.61 (m, 2H), 8.43 (d, $J$ = 8.4 Hz, 1H), 7.75 (d, $J$ = 1.2 Hz, 1H), 4.25 - 4.19 (m, 3H), 3.88 (br d, $J$ = 4.8 Hz, 3H), 3.86 - 3.84 (m, 3H), 3.70 (d, $J$ = 5.6 Hz, 2H), 3.56 (s, 3H), 1.83 (s, 3H), 1.79 (s, 3H), 1.76 - 1.70 (m, 1H), 1.08 - 1.01 (m, 2H), 0.96 - 0.88 (m, 2H).

[Example 12]

**Preparation of (4'-cyclopropyl-4-((4-(1-isopropyl-4-(trifluoromethyl)-1H-imidazol-2-yl)benzyl)amino)-6'-methoxy-[2,5'-bipyrimidine]-5-yl)dimethylphosphine oxide**

The titled compound was prepared from the starting material (4-(1-isopropyl-4-(trifluoromethyl)-1H-imidazol-2-yl)phenyl)methanamine by a method similar to the preparation method of Example 1 according to the following reaction scheme.

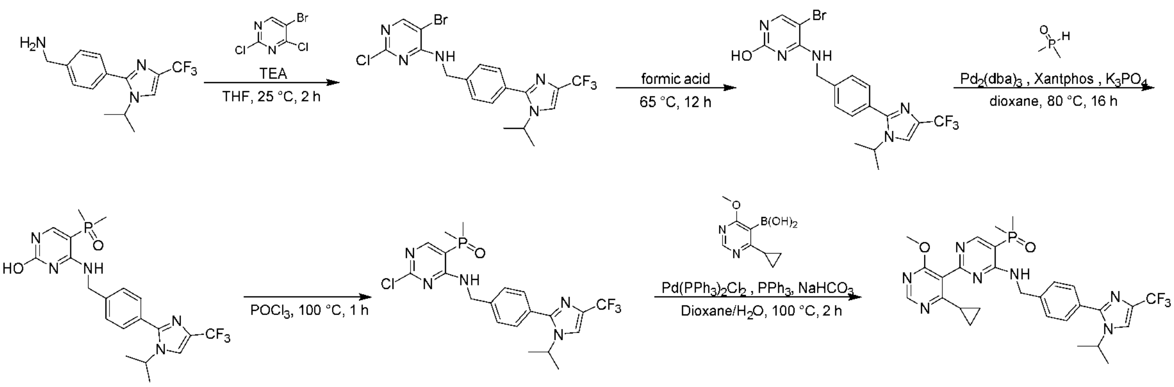


$^1H$ NMR (400 MHz, DMSO-$d_6$) $\delta$ = 9.00 (br s, 1H), 8.62 (s, 1H), 8.51 (d, $J$ = 8.4 Hz, 1H), 8.17 (d, $J$ = 0.8 Hz, 1H), 7.55 - 7.49 (m, 2H), 7.48 - 7.41 (m, 2H), 4.71 (br d, $J$ = 6.0 Hz, 2H), 4.45 (td, $J$ = 6.4, 13.2 Hz, 1H), 3.83 (s, 3H), 1.85 (d, $J$ = 13.6 Hz, 6H), 1.78 - 1.68 (m, 1H), 1.40 (d, $J$ = 6.4 Hz, 6H), 0.98 (td, $J$ = 3.6, 7.2Hz, 2H), 0.86 - 0.73 (m, 2H).

[Example 13]

**Preparation of (4'-cyclopropyl-4-((3-fluoro-4-(1-isopropyl-4-(trifluoromethyl)-1*H*-imidazol-2-yl)benzyl)amino)-6'-methoxy-[2,5'-bipyrimidine]-5-yl)dimethylphosphine oxide**

The titled compound was prepared from the starting material (3-fluoro-4-(1-isopropyl-4-(trifluoromethyl)-1H-imidazol-2-yl)phenyl)methanamine by a method similar to the preparation method of Example 1 according to the following reaction scheme.

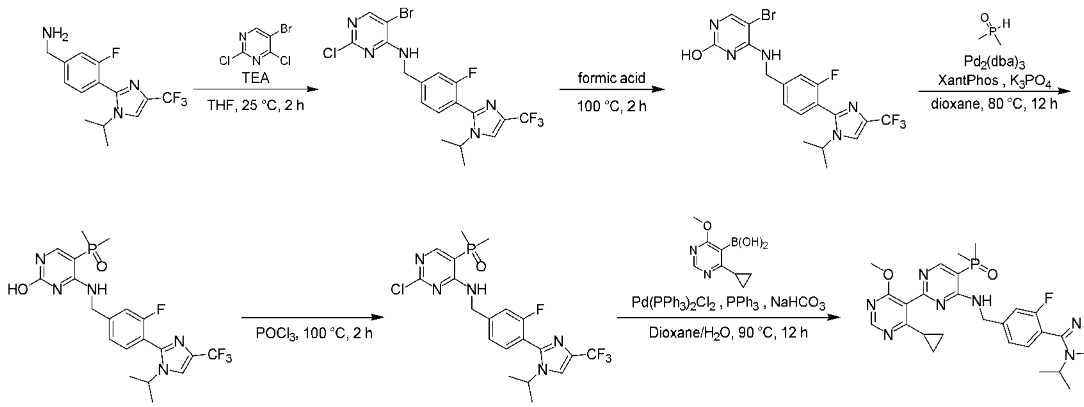


$^1$H NMR (400 MHz, DMSO-d6) δ = 9.01 (br t, J = 5.6 Hz, 1H), 8.62 (s, 1H), 8.53 (d, J = 8.4 Hz, 1H), 8.24 (s, 1H), 7.50 (t, J = 7.6 Hz, 1H), 7.37 - 7.24 (m, 2H), 4.72 (br d, J = 6.0 Hz, 2H), 4.12 (td, J = 6.4, 13.2 Hz, 1H), 3.82 (s, 3H), 1.86 (d, J = 13.6 Hz, 6H), 1.76 - 1.69 (m, 1H), 1.35 (d, J = 6.4 Hz, 6H), 0.99 (br d, J = 2.8 Hz, 2H), 0.81 (br dd, J = 3.2, 8.0 Hz, 2H).

[Example 14]

**Preparation of (4'-cyclopropyl-4-((3-fluoro-4-(5-methyl-3-(trifluoromethyl)-1*H*-pyrazole-1-yl)benzyl)amino)-6'-methoxy-[2,5'-bipyrimidine]-5-yl)dimethylphosphine oxide**

The titled compound was prepared from the starting material (3-fluoro-4-(5-methyl-3-(trifluoromethyl)-1H-pyrazole-1-yl)phenyl)methanamine by a method similar to the preparation method of Example 1 according to the following reaction scheme.

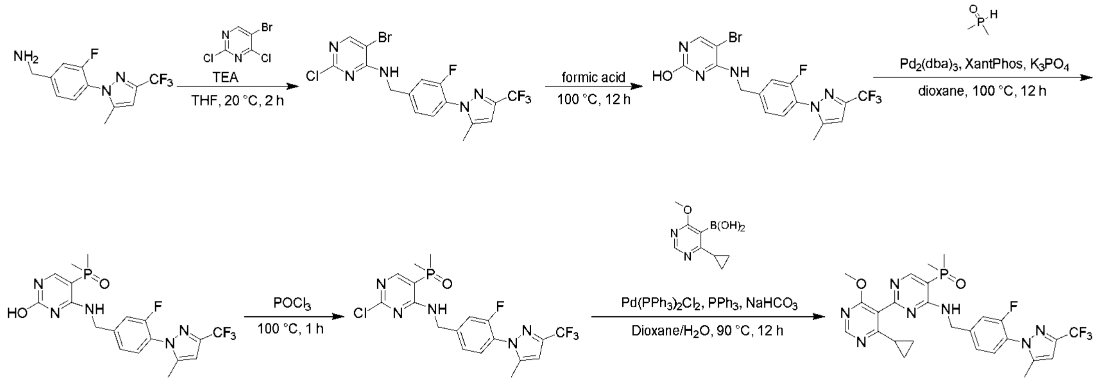


$^1$H NMR (400 MHz, DMSO-$d_6$) $\delta$ = 9.02 (br t, J= 6.0 Hz, 1H), 8.63 (s, 1H), 8.53 (d, *J* = 8.4 Hz, 1H), 7.59 (t, *J* = 8.0 Hz, 1H), 7.43 (dd, *J* = 1.2, 11.2 Hz, 1H), 7.32 (dd, *J* = 1.2, 8.0 Hz, 1H), 6.78 (s, 1H), 4.73 (d, *J* = 6.0 Hz, 2H), 3.82 (s, 3H), 2.19 (s, 3H), 1.86 (d, *J* = 13.6 Hz, 6H), 1.76 - 1.66 (m, 1H), 1.04 - 0.95 (m, 2H), 0.87 - 0.77 (m, 2H).

[Example 15]

**Preparation of (4'-cyclopropyl-4-((4-(5-isopropyl-3-(trifluoromethyl)-1*H*-pyrazole-1-yl)benzyl)amino)-6'-methoxy-[2,5'-bipyrimidine]-5-yl)dimethylphosphine oxide**

The titled compound was prepared from the starting material (4-(5-isopropyl-3-(trifluoromethyl)-1H-pyrazole-1-yl)phenyl)methanamine by a method similar to the preparation method of Example 1 according to the following reaction scheme.

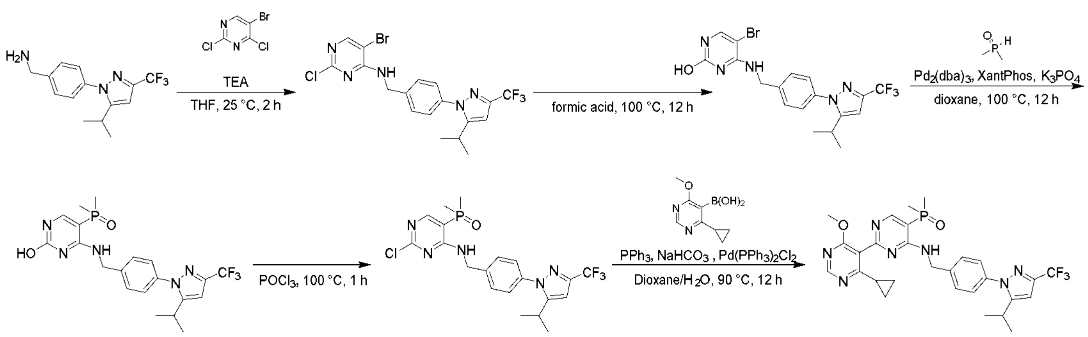


$^1$H NMR (400 MHz, DMSO-$d_6$) $\delta$ = 9.01 (br t, $J$ = 5.6 Hz, 1H), 8.62 (s, 1H), 8.52 (d, $J$ = 8.8 Hz, 1H), 7.48 (s, 4H), 6.81 (s, 1H), 4.73 (d, $J$ = 6.0 Hz, 2H), 3.83 (s, 3H), 2.94 (quin, $J$ = 6.8 Hz, 1H), 1.85 (d, $J$ = 13.6 Hz, 6H), 1.77 - 1.69 (m, 1H), 1.13 (d, $J$ = 6.8 Hz, 6H), 0.98 (quin, $J$ = 3.6 Hz, 2H), 0.86 - 0.77 (m, 2H).

[Example 16]

**Preparation of (4'-cyclopropyl-4-((3-fluoro-4-(5-isopropyl-3-(trifluoromethyl)-1*H*-pyrazol-1-yl)benzyl)amino)-6'-methoxy-[2,5'-bipyrimidine]-5-yl)dimethylphosphine oxide**

The titled compound was prepared from the starting material (3-fluoro-4-(5-isopropyl-3-(trifluoromethyl)-1H-pyrazole-1-yl)phenyl)methanamine by a method similar to the preparation method of Example 1 according to the following reaction scheme.

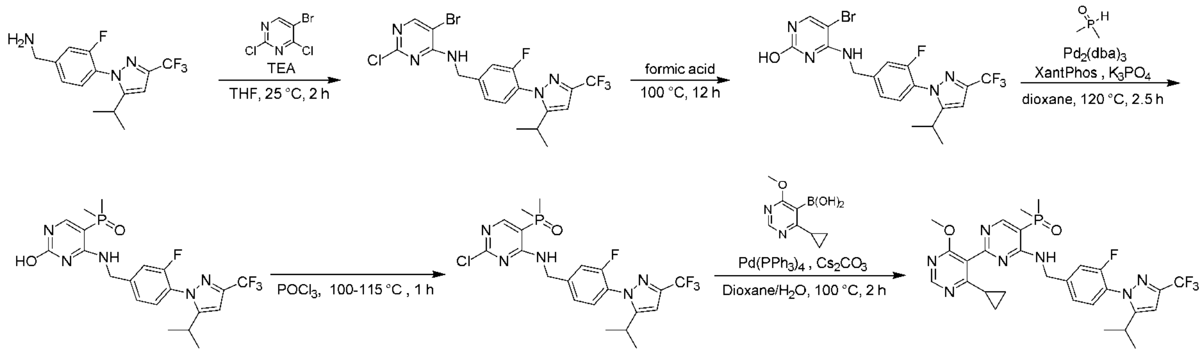


$^1$H NMR (400 MHz, DMSO-$d_6$) $\delta$ = 9.03 (br t, $J$ = 6.0 Hz, 1H), 8.62 (s, 1H), 8.53 (d, $J$ = 8.8 Hz, 1H), 7.61 (t, $J$ = 8.0 Hz, 1H), 7.43 (dd, $J$ = 1.2, 11.0 Hz, 1H), 7.32 (dd, $J$ = 1.2, 8.2 Hz, 1H), 6.85 (s, 1H), 4.74 (d, $J$ = 6.0 Hz, 2H), 3.82 (s, 3H), 2.69 (td, $J$ = 6.8, 13.6 Hz, 1H), 1.88 (s, 3H), 1.85 (s, 3H), 1.76 - 1.66 (m, 1H), 1.13 (s, 3H), 1.11 (s, 3H), 1.02 - 0.95 (m, 2H), 0.87 - 0.76 (m, 2H).

[Example 17]

**Preparation of (4'-cyclopropyl-4-((4-(5-cyclopropyl-3-(trifluoromethyl)-1*H*-pyrazole-1-yl)benzyl)amino)-6'-methoxy-[2,5'-bipyrimidine]-5-yl)dimethylphosphine oxide**

The titled compound was prepared from the starting material (4-(5-cyclopropyl-3-(trifluoromethyl)-1H-pyrazole-1-yl)phenyl)methanamine by a method similar to the preparation method of Example 1 according to the following reaction scheme.

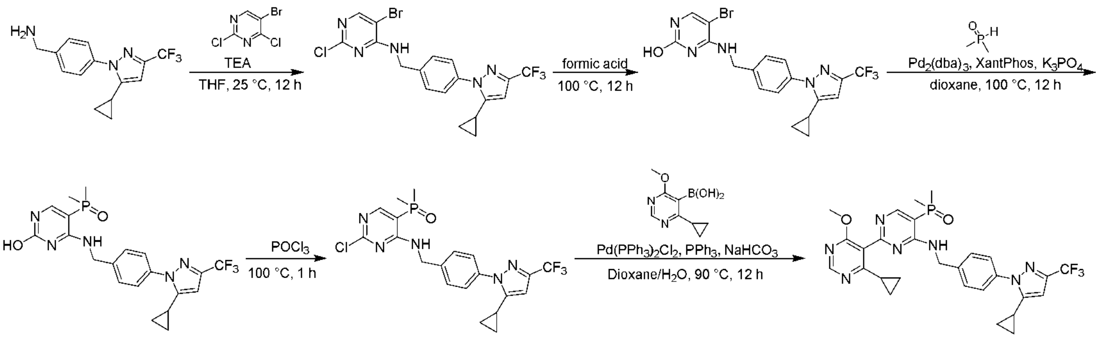


$^1H$ NMR (400 MHz, DMSO-$d_6$) $\delta$ = 8.99 (t, *J* = 6.0 Hz, 1H), 8.62 (s, 1H), 8.51 (d, *J* = 8.4 Hz, 1H), 7.60 (d, *J* = 8.4 Hz, 2H), 7.49 (d, *J* = 8.4 Hz, 2H), 6.62 (s, 1H), 4.72 (d, *J* = 6.0 Hz, 2H), 3.83 (s, 3H), 1.87 (s, 3H), 1.83 (s, 3H), 1.82 - 1.70 (m, 2H), 1.01 - 0.92 (m, 4H), 0.86 - 0.78 (m, 4H).

[Example 18]

**Preparation of (4'-cyclopropyl-4-((4-(5-cyclopropyl-3-(trifluoromethyl)-1*H*-pyrazole-1-yl)-3-fluorobenzyl)amino)-6'-methoxy[2,5'-bipyrimidine]-5-yl)dimethylphosphine oxide.**

The titled compound was prepared from the starting material (4-(5-cyclopropyl-3-(trifluoromethyl)-1H-pyrazole-1-yl)-3-fluorophenyl)methanamine by a method similar to the preparation method of Example 1 according to the following reaction scheme.

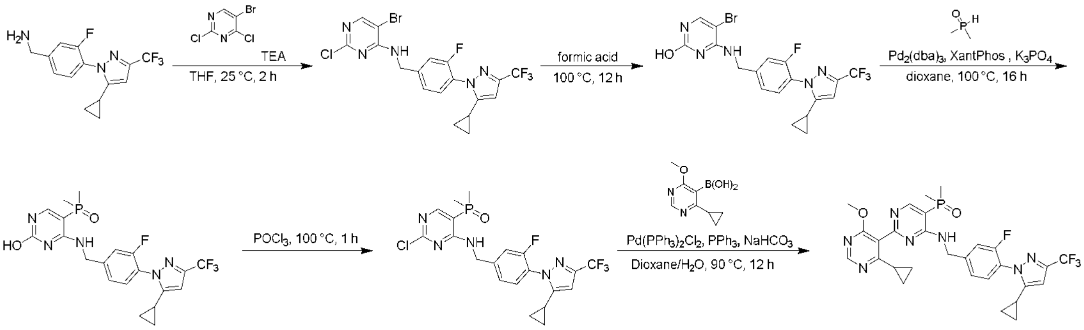


$^1H$ NMR (400 MHz, DMSO-$d_6$) $\delta$ = 8.67 - 8.56 (m, 2H), 7.64 (br t, *J* = 8.0 Hz, 1H), 7.46 (br d, *J* = 10.8 Hz, 1H), 7.35 (br d, *J* = 8.0 Hz, 1H), 7.15 (br d, *J* = 7.6 Hz, 1H), 6.62 (s, 1H), 4.69 (br d, *J* = 5.6 Hz, 2H), 3.82 (s, 3H), 1.80 - 1.72 (m, 1H), 1.60 (d, *J* = 13.6 Hz, 6H), 1.55 (br s, 1H), 0.99 (br s, 2H), 0.92 - 0.87 (m, 2H), 0.82 (br d, *J* = 5.2 Hz, 2H), 0.79 - 0.74 (m, 2H).

**[Experimental Example 1] Biochemical analysis of USP1 enzyme activity**

In this experimental example, the inhibitory effect of the compounds according to the present invention and the positive control compound on USP1 deubiquitinating activity was tested using a biochemical enzymatic assay. Ubiquitin-rhodamine 110 was used as a substrate for measuring deubiquitinating activity. This substrate compound is a substance in which rhodamine 110 is linked to the glycine residue at the C-terminus of ubiquitin by an amide bond. When this amide bond is cleaved by the deubiquitinating activity, a fluorescent signal is counted.

To obtain $IC_{50}$ values for biochemical analysis of the USP1 enzyme, 10 mM dimethyl sulfoxide (DMSO) stock solutions of the compounds to be tested were diluted to the final experimental concentration and 100 nL was added to each well of an analytical well plate. The compounds of the present invention were used as the compounds to be tested, and ML323 (*N*-(4-(1*H*-1,2,3-triazol-1-yl)benzyl)-5-methyl-2-(2-isopropylphenyl)pyrimidin-4-amine), known as a USP1 inhibitor, was used as the control compound.

Subsequently, analytical buffer solution (50 mM hydroxyethylpiperazineethanesulfonic acid (HEPES) containing USP1 enzyme, 100 mM NaCl, 2 mM tris(2-carboxyethyl)phosphine (TCEP), and 100 μg/mL bovine serum albumin (BSA)) were added to each well of the plate and incubated at room temperature for 10 minutes. Subsequently, analytical y buffer solution containing ubiquitin-rhodamine 110 was added to each well and reacted at room temperature for 30 minutes. The resulting fluorescence was measured using a PerkinElmer Envision instrument (excitation wavelength: 385 nm, emission wavelength: 535 nm). The measured values were analyzed using the XLfit model (205).

In this way, the concentration at which fluorescence of 50% of the maximum fluorescence intensity was observed for each compound was calculated as the half-inhibitory concentration ($IC_{50}$) and summarized in Table 1 below. $IC_{50}$ values were classified as A for less than 50 nM, B for the range of 50-100 nM, and C for more than 100 nM.

[Table 1]

| Compound | Enzyme activity IC50 grade | Compound | Enzyme activity IC50 grade |
|---|---|---|---|
| ML323 | C | Example10 | B |
| Example 1 | B | Example 11 | A |
| Example 2 | B | Example 12 | B |
| Example 3 | C | Example 13 | A |
| Example 4 | C | Example 14 | A |
| Example 5 | B | Example 15 | A |
| Example 6 | C | Example 16 | A |
| Example 7 | B | Example 17 | A |
| Example 8 | A | Example 18 | A |
| Example 9 | A | | |

**[Experimental Example 2] Cell survival analysis**

In this experimental example, the cell growth inhibitory effect of compounds according to the present invention and positive control compounds in tumor cell lines was examined.

MDA-MB-436 (ATCC, #HTB-130) cell line was cultured in cell culture medium (Leibovitz's L-15 (GIBCO catalog no. 11415064) + 10 μg/mL insulin (Yeasen catalog no. 40112ES25) + 16 μg/mL glutathione (GIBCO catalog no. 25030-081) + 10% fetal bovine serum (FBS) (Excell Bio catalog no. FSP500)). 3,000 MDA-MB-436 cells were plated per well in a 96-well plate and cultured for 1 day at 37°C in 5% $CO_2$. Using a 10-fold concentrated stock solution of the compound in dimethyl sulfoxide (DMSO) solvent, the compound, the control compound (ML323), or a blank sample of DMSO without the compound was added to each well to adjust the concentration of the compound in the culture medium to the final test concentration (0.25%). The cells were then cultured in a cell incubator for 10 days, and the cell culture medium containing the compound was replaced on the 5th day of culture. Cell survival rate was analyzed for luminescence using the Promega CellTiter-Glo Luminescent Cell Viability Assay Kit (Promega catalog number G7573), and the luminescence was measured using a PerkinElmer EnVision instrument. The percentage of cell growth inhibition rate was calculated from the measured relative luminescence unit (RLU) as follows.

$$Inhibition\ \ rate = 1 - \left( \frac{\text{RLU compound} - \text{RLU blank}}{\text{RLU control} - \text{RLU blank}} \right) \text{X } 100$$

From this cell growth inhibition rate, the half-maximal inhibitory concentration (IC50), which is the concentration at which each compound inhibits cell growth by 50%, was calculated using the GraphPad Prism program and was summarized in Table 2 below. IC50 values were classified as A for less than 100 nM, B for the range of 100-500 nM, and C for more than 500 nM.

[Table 2]

| Compound | Cell growth IC50 grade | Compound | Cell growth IC50 grade |
|---|---|---|---|
| ML323 | C | Example 10 | B |
| Example 1 | B | Example 11 | A |
| Example 2 | B | Example 12 | B |
| Example 3 | C | Example 13 | A |
| Example 4 | B | Example 14 | A |
| Example 5 | B | Example 15 | A |

(continued)

| Compound | Cell growth IC50 grade | Compound | Cell growth IC50 grade |
|---|---|---|---|
| Example 6 | B | Example 16 | A |
| Example 7 | A | Example 17 | A |
| Example 8 | A | Example 18 | A |
| Example 9 | A | | |

**[Experimental Example 3] BRCA1 synthetic lethality assay**

In this experimental example, whether the compound according to the present invention had synthetic lethal efficacy with BRCA1 activity was tested by examining the colony formation of tumor cells. MDA-MB-436 cells (ATCC, #HTB-130), a triple-negative breast cancer cell line with reduced BRCA1 activity due to BRCA1 mutation, and MCF-10A cell line (ATCC, #CRL-10317), which has wild-type BRCA1/2 genes and is used as a model of normal breast cells, were cultured in their respective culture media (MDA-MB-436: L-15 +10 mg/mL insulin + 16 mg/mL glutathione + 10% fetal bovine serum (FBS), MCF-10A: MEGM + 100 ng/mL cholera toxin), and then seeded in 6-well plates at a concentration of 10,000 cells per well for the MDA-MB-436 cells and 120 cells per well for the MCF-10A cell line. Next, the MDA-MB-436 cells were cultured for one day at 37°C under 100% air conditions, and the MCF-10A cells were cultured for one day at 37°C under 5% $CO_2$, 5% $CO_2$, and 95% air conditions. Then, a dimethyl sulfoxide (DMSO) solution of the compound of Example 1 was added to the plate in the form of a 10-fold concentrated solution of the final concentration (final concentration of DMSO solution: 0.1%). The culture medium was replaced every 7 days and cultured for 21 days. For colony formation analysis, the plate was washed with phosphate-buffered saline (PBS) on the 21st day, and the cells were fixed with PBS containing 4% paraformaldehyde for 15 minutes. After washing twice with distilled water, the cells were stained for 20 minutes at room temperature with a reagent containing 0.1% crystal violet in 10% ethanol. The cells stained with triple-distilled water were dried and imaged using a Chemi Doc MP imaging system. The crystal violet stained in the cells was extracted by treating the cells with 500 μL of 10% acetic acid for 20 minutes at room temperature for quantitative analysis. 150 μL of the extracted crystal violet was placed in two wells of a 96-well plate, and the absorbance was measured at 565 nm. The absorbance was quantified relative to the absorbance value of a blank sample containing only DMSO but not the compound of the present invention. The experimental results are summarized in Fig. 1.

As shown in the absorbance graph relative to DMSO control of A of Fig. 1, in MCF-10A cells with wild-type BRCA1/2, the crystal violet extraction amount was no different from that of the DMSO blank when compound of Example 1 was added. However, in MDA-MB-436 cells with reduced BRCA1 activity, the crystal violet extraction amount was less than one-fifth of that of the DMSO blank, when exposed to compound of Example 1. In addition, the cell colony formation shown in B of Fig. 1 was similar to that of the case treated with a DMSO blank sample, and MCF-10A cells formed colonies at a normal level even in the presence of the compound of the present invention, but colony formation of MDA-MB-436 cells was significantly reduced when the compound of the present invention was added.

From these results, it was found that the compound of the present invention has the selectivity to exhibit synthetic lethal efficacy through combination with BRCA1 activity.

While the present invention has been described through limited examples, the technical spirit and scope of the present invention are not limited to these examples, and the invention of the patent claims described below, as well as various modifications or variations of the invention described in the patent claims that are obvious to those skilled in the art to which this invention pertains, are also included within the scope of the present invention within the equivalent scope of the invention described in the patent claims.

## Claims

1. A compound of Chemical Formula 1 or a tautomer thereof and a pharmaceutically acceptable salt thereof:

[Chemical Formula 1]

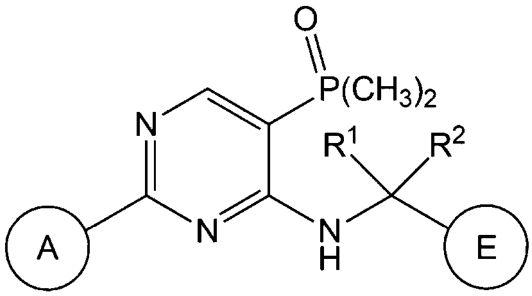

in Chemical Formula 1, $R^1$ and $R^2$ are each independently hydrogen, deuterium, halogen, $C_{1-4}$ alkyl, -O-$C_{1-4}$ alkyl, -CN or -NH$C_{1-4}$ alkyl;

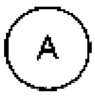 and 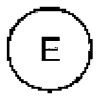

are rings each independently selected from the group consisting of substituted or non-substituted $C_{6-10}$ aryl, substituted or non-substituted 5- to 6-membered heteroaryl, substituted or non-substituted $C_{5-10}$ cycloalkyl and substituted or non-substituted cubanyl;
wherein the substituted 5- to 6-membered heteroaryl represents a ring in which at least one of the ring hydrogens is substituted with a functional group each independently selected from the group consisting of $C_{1-6}$ alkyl, $C_{1-6}$ alkyl substituted with deuterium, halo$C_{1-6}$ alkyl, $C_{3-8}$ cycloalkyl, -O$C_{1-4}$ alkyl, -O$C_{1-4}$ alkyl substituted with deuterium, halo$C_{1-4}$ alkyloxy and halogen,
wherein the substituted $C_{6-10}$ aryl, substituted $C_{5-10}$ cycloalkyl, substituted $C_{5-10}$ cycloalkyl and substituted cubanyl represent rings in which at least one of the ring hydrogens is substituted with a functional group each independently selected from the group consisting of $C_{1-6}$ alkyl, $C_{1-6}$ alkyl substituted with deuterium, halo$C_{1-6}$ alkyl, $C_{3-8}$cyclo alkyl, -O$C_{1-4}$ alkyl, -O$C_{1-4}$ alkyl substituted with deuterium, halo$C_{1-4}$ alkyloxy, halogen, 5- to 6-membered heteroaryl and functionality bearing 5- to 6-membered heteroaryl,
wherein the functionality bearing 5- to 6-membered heteroaryl represents a heteroaryl ring in which one or more of the ring hydrogens thereof is substituted with $C_{1-6}$ alkyl, halo$C_{1-6}$ alkyl or $C_{3-8}$ cycloalkyl.

**2.** The compound according to claim 1, wherein the compound or a tautomer thereof and a pharmaceutically acceptable salt thereof has a structure of Chemical Formula 2:

[Chemical Formula 2]

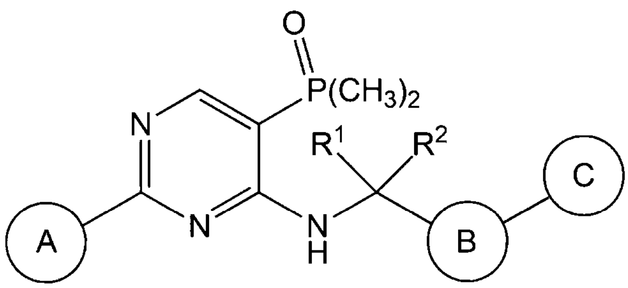

in Chemical Formula 2, $R^1$ and $R^2$ are each independently hydrogen, deuterium, halogen, $C_{1-4}$ alkyl, -O-$C_{1-4}$ alkyl, -CN or -NH$C_{1-4}$ alkyl;

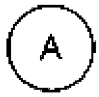

is selected from the group consisting of substituted or non-substituted $C_{6-10}$ aryl, substituted or non-substituted 5- to 6-membered heteroaryl, substituted or non-substituted $C_{5-10}$ cycloalkyl and substituted or non-substituted cubanyl;

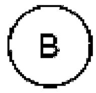

is selected from the group consisting of substituted or non-substituted phenylene, substituted or non-substituted 5- to 6-membered heteroarylene, substituted or non-substituted $C_{5-10}$ bicycloalkylene, substituted or non-substituted adamantylene, substituted or non-substituted norbornenyl and substituted or non-substituted cubanylene;

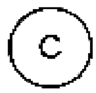

is selected from substituted or non-substituted phenyl and substituted or non-substituted 5- to 6-membered heteroarylene;
wherein the substituted $C_{6-10}$ aryl, substituted phenylene, substituted 5- to 6-membered heteroaryl, substituted 5- to 6-membered heteroarylene, substituted $C_{5-10}$ cycloalkyl, substitute $C_{5-10}$ bicycloalkyl, substituted $C_{5-10}$ bicycloalkylene, substituted adamantylene, substituted norbornenylene, substituted cubanyl, and substituted cubanylene represent that one or more hydrogen is substituted with a functional group each independently selected from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, -$OC_{1-4}$ alkyl substituted with deuterium, halo$C_{1-4}$ alkyloxy and halogen.

**3.** The compound according to claim 2, wherein

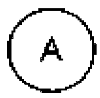

is substituted pyrimidine, substituted pyrazole, or substituted pyridine.

**4.** The compound according to claim 2, wherein the $C_{5-10}$ bicycloalkylene is bicyclo[1.1.1]pentanylene, bicyclo[2.2.1]heptanylene, bicyclo[2.1.1]hexanylene or bicyclo[2.2.2]octanylene.

**5.** The compound according to claim 2, wherein the compound or a tautomer thereof has a structure of Chemical Formula 3:

[Chemical Formula 3]

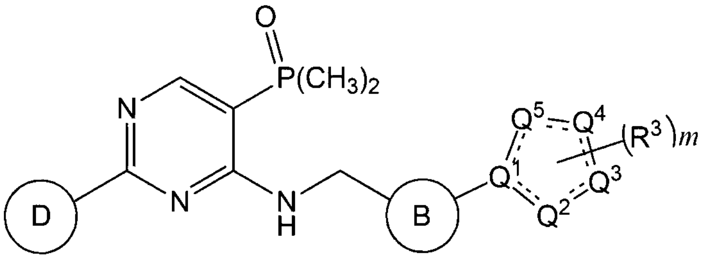

in Chemical Formula 3, $R^1$ and $R^2$ are each independently hydrogen, deuterium, halogen, $C_{1-4}$ alkyl, -O-$C_{1-4}$ alkyl, -CN or -NH$C_{1-4}$ alkyl;

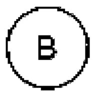

is selected from the group consisting of substituted or non-substituted phenylene, substituted or non-substituted 5- to 6-membered heteroarylene, substituted or non-substituted $C_{5-10}$ bicycloalkylene, substituted or non-substituted adamantylene, substituted or non-substituted norbornenyl and substituted or non-substituted cubanylene;

$Q^1$ to $Q^5$ are so selected from the group consisting of C, CH, N and NH as to form any one of the rings represented in Chemical Formula 4,

[Chemical Formula 4]

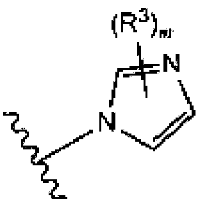 , 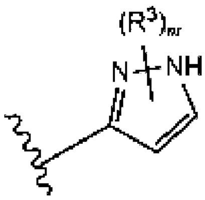 , 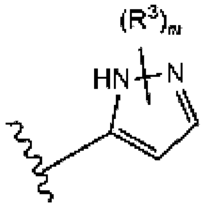 , 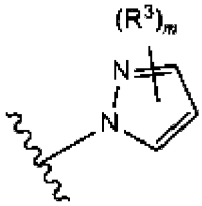 ,

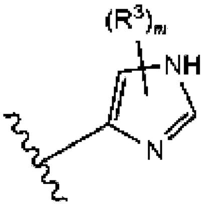 or 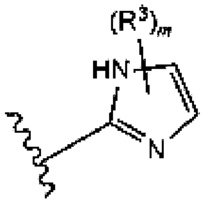

wherein m is an integer of 0 to 3, and $R^3$ is, in each case, a substituent independently selected for each occurrence leading up to the value of m, from the group consisting of $C_{1-6}$ alkyl, $C_{1-6}$ alkyl substituted with deuterium, halo$C_{1-6}$ alkyl, $C_{3-8}$ cycloalkyl, -O$C_{1-4}$ alkyl, -O$C_{1-4}$ alkyl substituted with deuterium, halo$C_{1-4}$ alkyloxy, and halogen,

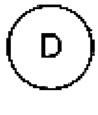

is a ring selected from Chemical Formula 5;

[Chemical Formula 5]

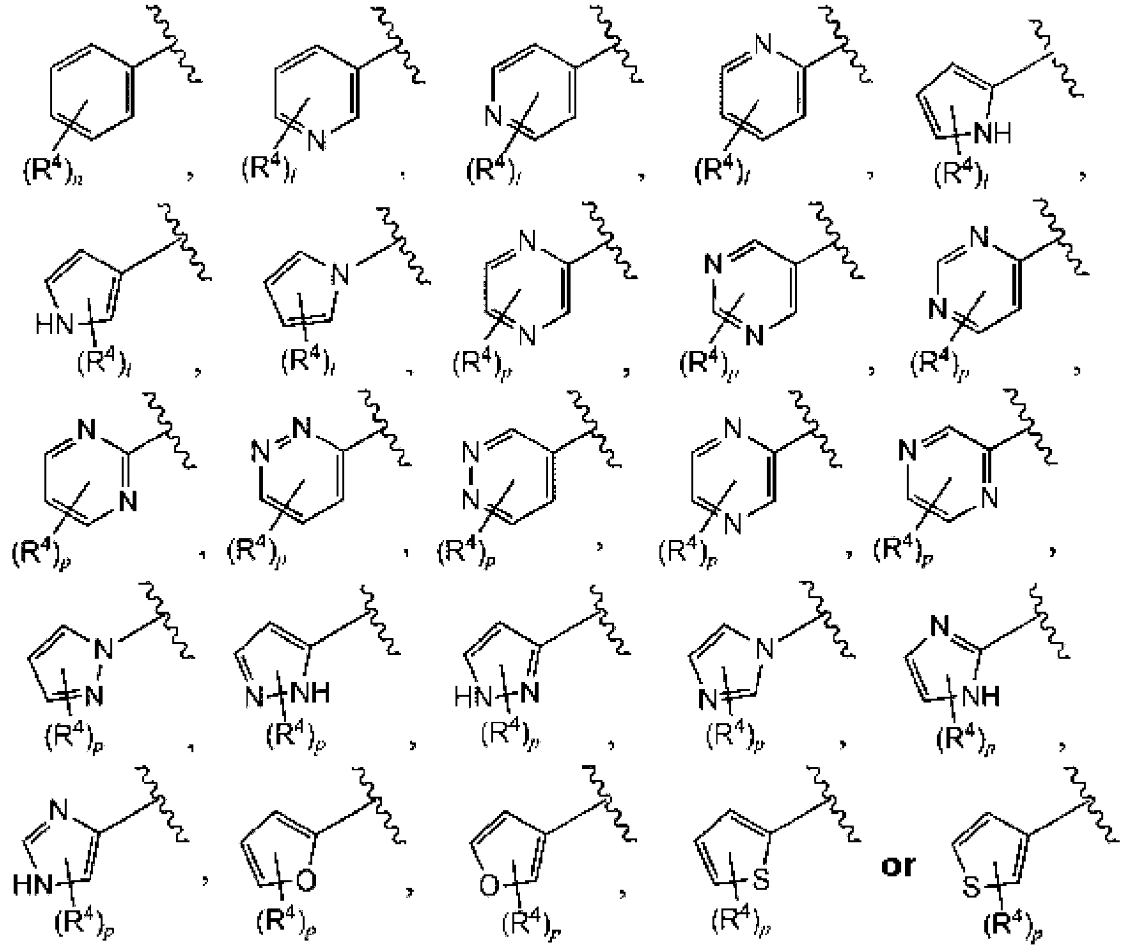

, , , , ,
, , , , ,
, , , , ,
, , , , ,
, , , or

wherein n is an integer of 0 to 5, *l* is an integer of 0 to 4, *p* is an integer of 0 to 3,
$R^4$ is, in each case, a substituent independently selected, for each occurrence leading up to the respective value of *n, l* or *p*, from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, -$OC_{1-4}$ alkyl substituted with deuterium, halo$C_{1-4}$ alkyloxy and halogen, and
wherein the substituted phenylene, substituted 5- to 6-membered heteroarylene, substituted $C_{5-10}$ bicycloalkylene, substituted adamantylene, substituted norbornenyl and substituted cubanylene represent that one or more hydrogen is substituted with a functional group each independently selected from the group consisting of $C_{1-6}$ alkyl, $C_{1-6}$ alkyl substituted with deuterium, halo$C_{1-6}$ alkyl, $C_{3-8}$ cycloalkyl, -$OC_{1-4}$ alkyl, -$OC_{1-4}$ alkyl substituted with deuterium, halo$C_{1-4}$ alkyloxy and halogen.

**6.** The compound according to claim 5, wherein

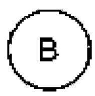

is non-substituted phenylene or phenylene substituted with halogen.

**7.** The compound according to claim 5, wherein the compound or tautomer thereof has a structure of Chemical Formula 6:

[Chemical Formula 6]

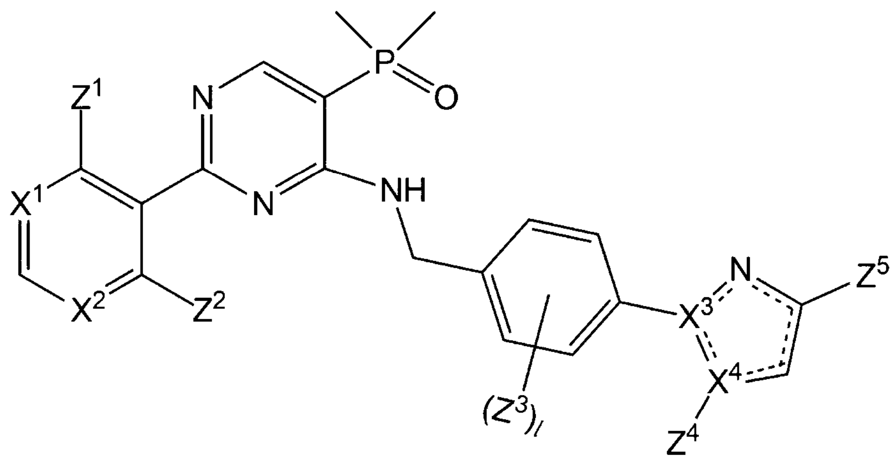

in Chemical Formula 6, $X^1$ and $X^2$ are each independently CH or N;
$X^3$ and $X^4$ are each independently C or N;
$Z^1$, $Z^2$, $Z^4$ and $Z^5$ are each independently selected from the group consisting of hydrogen, $C_{1-6}$ alkyl, $C_{1-6}$ alkyl substituted with deuterium, halo$C_{1-6}$ alkyl, $C_{3-8}$ cycloalkyl, -O$C_{1-4}$ alkyl, -O$C_{1-4}$ alkyl substituted with deuterium, halo$C_{1-4}$ alkyloxyl and halogen,
$Z^3$ is a substituent each independently selected at each occurrence up to *l* times from the group consisting of $C_{1-6}$ alkyl, $C_{1-6}$ alkyl substituted with deuterium, halo$C_{1-6}$ alkyl, $C_{3-8}$ cycloalkyl, -O$C_{1-4}$alkyl, -O$C_{1-4}$ alkyl substituted with deuterium, halo$C_{1-4}$ alkyloxy and halogen, and
*l* is an integer of 0 to 4.

**8.** The compound according to claim 7, wherein $Z^3$ is hydrogen or halogen, and $Z^4$ and $Z^5$ are each independently hydrogen, $C_{1-6}$ alkyl, $C_{3-8}$ cycloalkyl or halo$C_{1-6}$ alkyl.

**9.** A compound selected from the group consisting of the following compounds, stereoisomer, tautomer, solvate or a pharmaceutically acceptable salt thereof:

(2-(2-cyclopropyl-6-methoxyphenyl)-4-((4-(1-methyl-4-(trifluoromethyl)-1*H*-imidazol-2-yl)benzyl)amino)pyrimidin-5-yl)dimethylphosphine oxide;
(4'-cyclopropyl-6'-methoxy-4-((4-(1-methyl-4-(trifluoromethyl)-1*H*-imidazol-2-yl)benzyl)amino)-[2,5'-bipyrimidine]-5-yl)dimethylphosphine oxide;
(2-(1-isopropyl-4-methyl-1H-pyrazole-5-yl)-4-((4-(1-methyl-4-(trifluoromethyl)-1*H*-imidazol-2-yl)benzyl)amino) pyrimidin-5-yl)dimethylphosphine oxide;
(2-(2-isopropylpyridine-3-yl)-4-((4-(1-methyl-4-(trifluoromethyl)-1*H*-imidazol-2-yl)benzyl)amino)pyrimidin-5-yl) dimethylphosphine oxide;
(4'-cyclopropyl-6'-(methoxy-$d_3$)-4-((4-(1-methyl-4-(trifluoromethyl)-1*H*-imidazol-2-yl)benzyl)amino)-[2,5'-bipyrimidine]-5-yl)dimethylphosphine oxide;
(2-(2-(difluoromethoxy)pyridine-3-yl)-4-((4-(1-methyl-4-(trifluoromethyl)-1*H*-imidazol-2-yl)benzyl)amino)pyrimidin-5-yl)dimethylphosphine oxide;
(4'-cyclopropyl-4-((3-fluoro-4-(1-methyl-4-(trifluoromethyl)-1H-imidazol-2-yl)benzyl)amino)-6'-methoxy-[2,5'-bipyrimidine]-5-yl)dimethylphosphine oxide;
(4'-cyclopropyl-4-((4-(1-cyclopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-6'-methoxy-4-((4-(5-methyl-3-(trifluoromethyl)-1*H*-pyrazole-1-yl)benzyl)amino)-[2,5'-bipyrimidine]-5-yl)dimethylphosphine oxide;
(4'-cyclopropyl-6'-methoxy-4-(((4-(1-methyl-4-(trifluoromethyl)-1*H*-imidazol-2-yl)bicyclo[2.2.2]octan-1-yl) methyl)amino)-[2,5'-bipyrimidine]-5-yl)dimethylphosphine oxide;
(4'-cyclopropyl-6'-methoxy-4-((((1*s*,2*R*,3*s*,4*r*,5*S*,6*r*,7*R*,8*S*)-4-(1-methyl-4-(trifluoromethyl)-1*H*-imidazol-2-yl) cuban-1-yl)methyl)amino)-[2,5'-bipyrimidine]-5-yl)dimethylphosphine oxide;
(4'-cyclopropyl-4-((4-(1-isopropyl-4-(trifluoromethyl)-1*H*-imidazol-2-yl)benzyl)amino)-6'-methoxy-[2,5'-bipyrimidine]-5-yl)dimethylphosphine oxide;

(4'-cyclopropyl-4-((3-fluoro-4-(1-isopropyl-4-(trifluoromethyl)-1*H*-imidazol-2-yl)benzyl)amino)-6'-methoxy-[2,5'-bipyrimidine]-5-yl)dimethylphosphine oxide;
(4'-cyclopropyl-4-((3-fluoro-4-(5-methyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((3-fluoro-4-(5-isopropyl-3-(trifluoromethyl)-1*H*-pyrazole-1-yl)benzyl)amino)-6'-methoxy-[2,5'-bipyrimidine]-5-yl)dimethylphosphine oxide;
(4'-cyclopropyl-4-((4-(5-cyclopropyl-3-(trifluoromethyl)-1*H*-pyrazole-1-yl)benzyl)amino)-6'-methoxy-[2,5'-bipyrimidine]-5-yl)dimethylphosphine oxide; and
(4'-cyclopropyl-4-((4-(5-cyclopropyl-3-(trifluoromethyl)-1*H*-pyrazole-1-yl)-3-fluorobenzyl)amino)-6'-methoxy [2,5'-bipyrimidine]-5-yl)dimethylphosphine oxide.

**10.** A pharmaceutical composition for treating cancer, comprising a therapeutically effective amount of the compound according to claim 1 and a pharmaceutically acceptable excipient.

【FIG. 1】

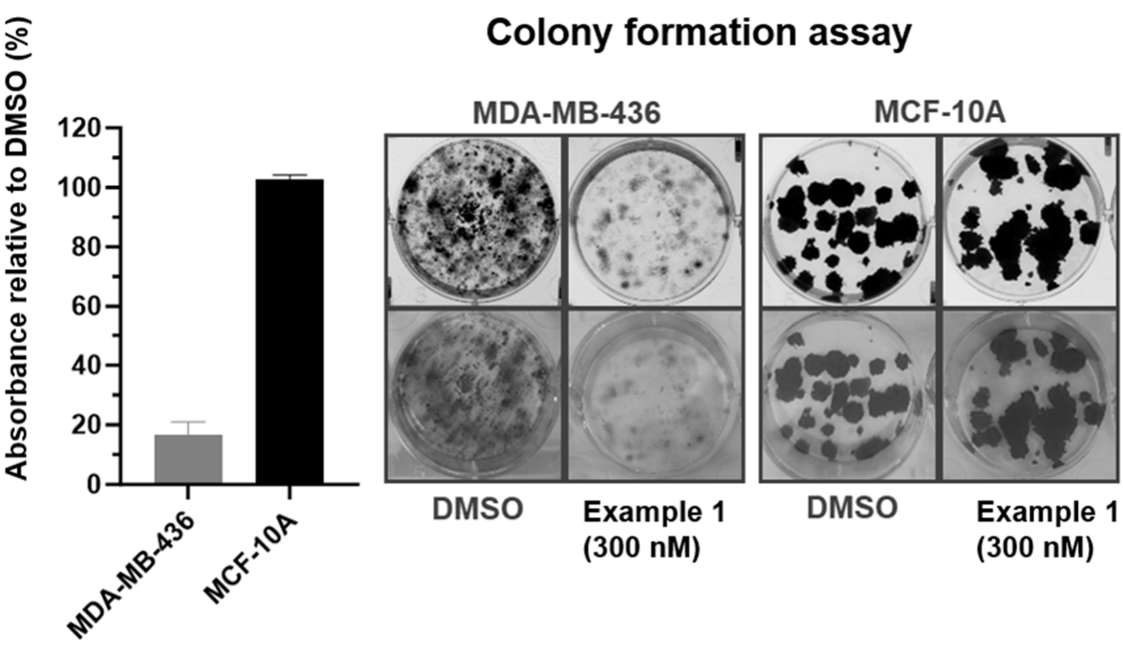
Absorbance relative to DMSO (%)
120
100
80
60
40
20
0
MDA-MB-436
MCF-10A
Colony formation assay
MDA-MB-436
MCF-10A
DMSO
Example 1 (300 nM)
DMSO
Example 1 (300 nM)

**INTERNATIONAL SEARCH REPORT**

International application No.
**PCT/KR2024/009140**

**A. CLASSIFICATION OF SUBJECT MATTER**

**C07D 421/12**(2006.01)i; **A61K 31/095**(2006.01)i; **A61K 31/517**(2006.01)i; **A61K 31/5377**(2006.01)i; **A61K 31/675**(2006.01)i; **A61K 31/695**(2006.01)i; **C07D 487/04**(2006.01)i; **C07D 471/04**(2006.01)i; **C07D 491/056**(2006.01)i; **C07D 421/14**(2006.01)i

According to International Patent Classification (IPC) or to both national classification and IPC

**B. FIELDS SEARCHED**

Minimum documentation searched (classification system followed by classification symbols)

C07D 421/12(2006.01); C07D 409/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus, Marpat) & keywords: 포스핀 유도체 (phosphine derivatives), 유비퀴틴 (ubiquitine), 암 (cancer)

**C. DOCUMENTS CONSIDERED TO BE RELEVANT**

| Category* | Citation of document, with indication, where appropriate, of the relevant passages | Relevant to claim No. |
|---|---|---|
| A | LIANG, Q. et al. A selective USP1–UAF1 inhibitor links deubiquitination to DNA damage responses. Nature Chemical Biology. 2014, vol. 10, pp. 298-304. See abstract; and figure 1. | 1-10 |
| A | DEXHEIMER, T. S. et al. Synthesis and structure–activity relationship studies of N-benzyl-2-phenylpyrimidin-4-amine derivatives as potent USP1/UAF1 deubiquitinase inhibitors with anticancer activity against nonsmall cell lung cancer. Journal of Medicinal Chemistry. 2014, vol. 57, pp. 8099-8110. See abstract; figure 1; and tables 1-3. | 1-10 |
| A | HUANG, W. -S. et al. Discovery of brigatinib (AP26113), a phosphine oxide-containing, potent, orally active inhibitor of anaplastic lymphoma kinase. Journal of Medicinal Chemistry. 2016, vol. 59, pp. 4948-4964. See abstract; and figures 1 and 2. | 1-10 |
| A | WO 2014-105952 A2 (THE UNITED STATES OF AMERICA, AS REPRESENTED BY THE SECRETARY, DEPARTMENT OF HEALTH AND HUMAN SERVICES et al.) 03 July 2014 (2014-07-03) See claims 1-30. | 1-10 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2024** | **10 October 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/009140**

**C. DOCUMENTS CONSIDERED TO BE RELEVANT**

| Category* | Citation of document, with indication, where appropriate, of the relevant passages | Relevant to claim No. |
|---|---|---|
| A | WO 2023-107603 A1 (KINETA, INC.) 15 June 2023. See claims 1-99. | 1-10 |
| PX | WO 2024-094170 A1 (SHENZHEN JINGTAI TECHNOLOGY CO., LTD.) 10 May 2024. See compounds 8 and 11; claims 1-25; and page 98. | 1-10 |

Form PCT/ISA/210 (second sheet) (July 2022)

**INTERNATIONAL SEARCH REPORT**
**Information on patent family members**

International application No.
**PCT/KR2024/009140**

| Patent document cited in search report | Publication date (day/month/year) | Patent family member(s) | Publication date (day/month/year) |
| --- | --- | --- | --- |
| WO 2014-105952 A2 | 03 July 2014 | AU 2013-370417 A1 | 16 July 2015 |
| | | CA 2896731 A1 | 03 July 2014 |
| | | EP 2938610 A2 | 04 November 2015 |
| | | JP 2016-504365 A | 12 February 2016 |
| | | US 2015-0344443 A1 | 03 December 2015 |
| | | US 9802904 B2 | 31 October 2017 |
| | | WO 2014-105952 A3 | 09 October 2014 |
| WO 2023-107603 A1 | 15 June 2023 | AU 2024-407039 A1 | 25 July 2024 |
| | | CA 3240381 A1 | 15 June 2023 |
| WO 2024-094170 A1 | 10 May 2024 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5846514 A
- US 6334997 B


### Non-patent literature cited in the description


- **COHN et al.** *Mol Cell*, 2007, vol. 28, 786-797
- **BERGE et al.** *J. Pharmaceutical Sciences*, 1977, vol. 66, 1-19
- **LOUIS F. FIESER** ; **MARY FIESER**. Reagents for Organic Synthesis. Wiley, 1967
- Beilsteins Handbuch der organischen Chemie. Springer-Verlag, vol. 4
- Recent Advances in the Synthesis and Applications of Radiolabeled Compounds for Drug Discovery and Development. Curr., Pharm. Des.. 2000, vol. 6